# EUROPEAN PATENT SPECIFICATION

(11) **EP 4 188 295 B1**
(45) Date of publication and mention of the grant of the patent: **30.10.2024**
(21) Application number: 21746164.9
(22) Date of filing: 20.07.2021
(51) Int. Cl.: A61F 13/00

(54) **UNENCAPSULATED OPEN-CAVITY DRESSING FOR NEGATIVE-PRESSURE THERAPY**
NICHT VERKAPSELTER OPEN-CAVITY-VERBAND FÜR DIE UNTERDRUCKTHERAPIE
PANSEMENT À CAVITÉ OUVERTE NON ENCAPSULÉ POUR THÉRAPIE PAR PRESSION NÉGATIVE

(30) Priority: 30.07.2020 US 202063058801 P
(43) Date of publication of application: 07.06.2023
(73) Proprietor: Solventum Intellectual Properties Company, Maplewood, MN 55144 (US)
(72) Inventor: LOCKE, Christopher Brian, Athlone, Co. Westmeath, N37XF22 (IE)
(74) Representative: Simmons & Simmons
(86) International application number: PCT/IB2021/056539
(87) International publication number: WO 2022/023877

(56) References cited:
- EP-A1- 2 340 066
- EP-B1- 2 340 066
- US-A1- 2020 060 879
- US-A1- 2020 170 842

## Description

### CROSS-REFERENCE TO RELATED APPLICATIONS

This application claims the benefit of priority to U.S. Provisional Application No. 63/058,801, filed on July 30, 2020.

### TECHNICAL FIELD

This disclosure relates generally to medical treatment systems and, more particularly, but not by way of limitation, to absorbent dressings, systems for treating a tissue site with reduced pressure.

### BACKGROUND

Clinical studies and practice have shown that reducing pressure in proximity to a tissue site can augment and accelerate growth of new tissue at the tissue site. The applications of this phenomenon are numerous, but have proven particularly advantageous for treating wounds. Regardless of the etiology of a wound, whether trauma, surgery, or another cause, proper care of a wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," and "vacuum-assisted closure," for example. Negative-pressure therapy may provide a number of benefits, including migration of epithelial and subcutaneous tissues, improved blood flow, removal of fluids (such as exudate) from a wound site, and micro-deformation of tissue at the wound site. Together, these benefits can increase development of granulation tissue and reduce healing times.

While the clinical benefits of negative-pressure therapy are widely known, the cost and complexity of negative-pressure therapy can be a limiting factor in its application, and the development and operation of negative-pressure systems, components, and processes continues to present significant challenges to manufacturers, healthcare providers, and patients.

EP2340366 discloses an open-cavity reduced pressure treatment system having an encapsulated treatment manifold with flow channels formed by open cells.

US2020/0170842 discloses a dressing with first and second liquid-impermeable layers fenestrate d layers forming a chamber, and a third layer disposed therebetween.

US2020/0060879 disclose a low-profile dressing interface with side-by-side fluid pathways.

### SUMMARY

The invention is defined by the appended independent claim. A selection of optional features is set out in the dependent claims.

### BRIEF DESCRIPTION OF THE DRAWINGS

FIGURE 1 is a block diagram of an example embodiment of a therapy system and a dressing that can provide negative-pressure treatment and instillation treatment in accordance with this specification;
FIGURE 2A is an isometric view of an example of a tissue interface that can be associated with some embodiments of the therapy system and the dressing of FIGURE 1;
FIGURE 2B is an exploded isometric view of an example of a tissue interface that can be associated with some embodiments of the therapy system and the dressing of FIGURE 1;
FIGURE 3A is a top view of the tissue interface of FIGURE 2A;
FIGURE 3B is a detail view of the tissue interface of FIGURE 3A taken at reference FIGURE 3B in FIGURE 3A;
FIGURE 4 is a top view of an example of a tissue interface under negative pressure;
FIGURE 5A is a top view of another example of a tissue interface that can be associated with some embodiments of the therapy system and the dressing of FIGURE 1;
FIGURE 5B is an isometric view of the tissue interface of FIGURE 5A, illustrating additional features that may be associated with some embodiments;
FIGURE 6A is an isometric view of another example of a tissue interface that can be associated with the therapy system and the dressing of FIGURE 1;
FIGURE 6B is an isometric view of a second side of the tissue interface of FIGURE 6A, illustrating additional features that may be associated with some embodiments;
FIGURE 7 is an isometric view of another example of a tissue interface that can be associated with the therapy system and the dressing of FIGURE 1;
FIGURE 8 is an isometric view, with a portion shown in cross-section, of an example tissue site;
FIGURE 9 is an isometric view, with a portion shown in cross-section, of a portion of an example embodiment of a therapy system and a dressing being deployed over an example tissue site;
FIGURE 10 is a table illustrating an improved percentage of collapse of a tissue interface according to this disclosure;
FIGURE 11A is a schematic diagram, with a portion in cross-section, of an illustrative example embodiment of an open-cavity, reduced-pressure treatment device within a therapy system similar to that of FIGURE 1;
FIGURE 11B is a schematic cross-section of a portion of the example treatment device of FIGURE 11A;
FIGURE 11C is a schematic cross-section of a portion of the example treatment device of FIGURE 11A taken along line 11C-11C;
FIGURE 11D is a schematic cross-section of a portion of the example system of FIGURE 11A;
FIGURE 12 is a schematic, isometric view of the example open-cavity, reduced-pressure treatment device of FIGURES 11A-11D;
FIGURE 13A is a schematic, plan view of another illustrative example embodiment of an open-cavity, reduced-pressure treatment device;
FIGURE 13B is a schematic, plan view of a portion of the example treatment device of FIGURE 13A;
FIGURE 13C is a schematic cross-section of a portion of the example treatment device of FIGURE 13B taken along line 13C-13C;
FIGURE 14A is a top plan view of yet another illustrative example of a treatment device that can be associated with some embodiments of the therapy system of FIGURES 1 and/or 11A;
FIGURE 14B is a detail isometric view of a portion of the treatment device of FIGURE 14A, illustrating additional details that may be associated with some embodiments;
FIGURE 14C is an exploded cross-sectional view of the leg portion of FIGURE 14B, illustrating additional details that may be associated with some embodiments;
FIGURE 14D is a schematic partial cross-section view illustrating the leg manifold member portion of FIGURE 14C, illustrating additional details that may be associated with some embodiments;
FIGURE 14E is a schematic partial cross-section view of another example embodiment of the leg manifold member, illustrating additional details that may be associated with some embodiments; and
FIGURE 14F is a partial isometric view of the treatment device of FIGURE 14A, illustrating additional details that may be associated with some embodiments.

### DETAILED DESCRIPTION OF ILLUSTRATIVE EMBODIMENTS

The following description of example embodiments enables a person skilled in the art to make and use the subject matter set forth in the appended claims. Certain details already known in the art may be omitted. Therefore, the following detailed description is illustrative and non-limiting.

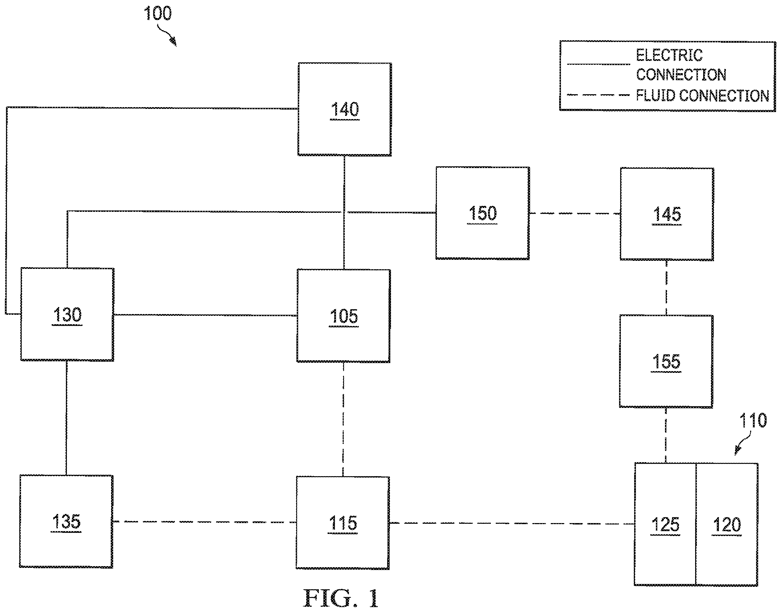

FIGURE 1 is a block diagram of an example embodiment of a therapy system 100 that can provide negative-pressure therapy with instillation of topical treatment solutions to a tissue site in accordance with this specification.

The term "tissue site" in this context broadly refers to a wound, defect, or other treatment target located on or within tissue, including but not limited to, a surface wound, bone tissue, adipose tissue, muscle tissue, neural tissue, dermal tissue, vascular tissue, connective tissue, cartilage, tendons, or ligaments. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted. A surface wound, as used herein, is a wound on a body that is exposed to the external environment, such as an injury or damage to the epidermis, dermis, and/or subcutaneous layers. Surface wounds may include ulcers or closed incisions, for example. A surface wound, as used herein, does not include wounds within an intra-abdominal cavity. A wound may include chronic, acute, traumatic, subacute, and dehisced wounds, partial-thickness burns, ulcers (such as diabetic, pressure, or venous insufficiency ulcers), flaps, and grafts, for example.

The therapy system 100 may include a source or supply of negative pressure, such as a negative-pressure source 105, and one or more distribution components. A distribution component is preferably detachable and may be disposable, reusable, or recyclable. A dressing, such as a dressing 110, and a fluid container, such as a container 115, are examples of distribution components that may be associated with some examples of the therapy system 100. As illustrated in the example of FIGURE 1, the dressing 110 may include a tissue interface 120, a cover 125, or both in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 110. For example, such a dressing interface may be a SENSAT.R.A.C.^{™} Pad available from Kinetic Concepts, Inc. of San Antonio, Texas.

The therapy system 100 may also include a regulator or controller, such as a controller 130. Additionally, the therapy system 100 may include sensors to measure operating parameters and provide feedback signals to the controller 130 indicative of the operating parameters. As illustrated in FIGURE 1, for example, the therapy system 100 may include a first sensor 135 and a second sensor 140 coupled to the controller 130.

The therapy system 100 may also include a source of instillation solution. For example, a solution source 145 may be fluidly coupled to the dressing 110, as illustrated in the example embodiment of FIGURE 1. The solution source 145 may be fluidly coupled to a positive-pressure source, such as a positive-pressure source 150, a negative-pressure source such as the negative-pressure source 105, or both in some embodiments. A regulator, such as an instillation regulator 155, may also be fluidly coupled to the solution source 145 and the dressing 110 to ensure proper dosage of instillation solution (e.g. saline) to a tissue site. For example, the instillation regulator 155 may comprise a piston that can be pneumatically actuated by the negative-pressure source 105 to draw instillation solution from the solution source during a negative-pressure interval and to instill the solution to a dressing during a venting interval. Additionally or alternatively, the controller 130 may be coupled to the negative-pressure source 105, the positive-pressure source 150, or both, to control dosage of instillation solution to a tissue site. In some embodiments, the instillation regulator 155 may also be fluidly coupled to the negative-pressure source 105 through the dressing 110, as illustrated in the example of FIGURE 1.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 105 may be combined with the controller 130, the solution source 145, and other components into a therapy unit.

In general, components of the therapy system 100 may be coupled directly or indirectly. For example, the negative-pressure source 105 may be directly coupled to the container 115 and may be indirectly coupled to the dressing 110 through the container 115. Coupling may include fluid, mechanical, thermal, electrical, or chemical coupling (such as a chemical bond), or some combination of coupling in some contexts. For example, the negative-pressure source 105 may be electrically coupled to the controller 130 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 105, may be a reservoir of air at a negative pressure or may be a manual or electrically-powered device, such as a vacuum pump, a suction pump, a wall suction port available at many healthcare facilities, or a micro-pump, for example. "Negative pressure" or "reduced pressure" (which may be used interchangeably) generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 105 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa), or between -100 mm Hg and -200 mm Hg.

The reduced pressure delivered may be constant, varied, patterned, or random, and may be delivered continuously or intermittently. Although the terms "vacuum" and "negative pressure" may be used to describe the pressure applied to the tissue site, the actual pressure applied to the tissue site may be more than the pressure normally associated with a complete vacuum. Consistent with the use herein, an increase in reduced pressure corresponds to a reduction in pressure (more negative relative to ambient pressure) and a decrease in reduced pressure corresponds to an increase in pressure (less negative relative to ambient pressure).

The container 115 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 130, may be a microprocessor or computer programmed to operate one or more components of the therapy system 100, such as the negative-pressure source 105. In some embodiments, for example, the controller 130 may be a microcontroller, which generally comprises an integrated circuit containing a processor core and a memory programmed to directly or indirectly control one or more operating parameters of the therapy system 100. Operating parameters may include the power applied to the negative-pressure source 105, the pressure generated by the negative-pressure source 105, or the pressure distributed to the tissue interface 120, for example. The controller 130 is also preferably configured to receive one or more input signals, such as a feedback signal, and programmed to modify one or more operating parameters based on the input signals.

Sensors, such as the first sensor 135 and the second sensor 140, are generally known in the art as any apparatus operable to detect or measure a physical phenomenon or property, and generally provide a signal indicative of the phenomenon or property that is detected or measured. For example, the first sensor 135 and the second sensor 140 may be configured to measure one or more operating parameters of the therapy system 100. In some embodiments, the first sensor 135 may be a transducer configured to measure pressure in a pneumatic pathway and convert the measurement to a signal indicative of the pressure measured. In some embodiments, for example, the first sensor 135 may be a piezo-resistive strain gauge. The second sensor 140 may optionally measure operating parameters of the negative-pressure source 105, such as a voltage or current, in some embodiments. Preferably, the signals from the first sensor 135 and the second sensor 140 are suitable as an input signal to the controller 130, but some signal conditioning may be appropriate in some embodiments. For example, the signal may need to be filtered or amplified before it can be processed by the controller 130. Typically, the signal is an electrical signal, but may be represented in other forms, such as an optical signal.

The tissue interface 120 can be generally adapted to partially or fully contact a tissue site. The tissue interface 120 may take many forms, and may have many sizes, shapes, or thicknesses, depending on a variety of factors, such as the type of treatment being implemented or the nature and size of a tissue site. For example, the size and shape of the tissue interface 120 may be adapted to the contours of deep and irregular shaped tissue sites. Any or all of the surfaces of the tissue interface 120 may have an uneven, coarse, or jagged profile.

In some embodiments, the tissue interface 120 may include or may be a manifold. A manifold in this context may include a means for collecting or distributing fluid across or through the tissue interface 120 under pressure. For example, a manifold may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed or a secondary fluid path may be provided to facilitate delivering fluid, such as fluid from a source of instillation solution, across a tissue site.

In some embodiments, the cover 125 may provide a bacterial barrier and protection from physical trauma. The cover 125 may also be constructed from a material that can reduce evaporative losses and provide a fluid seal between two components or two environments, such as between a therapeutic environment and a local external environment. The cover 125 may be, for example, an elastomeric film or membrane that can provide a seal adequate to maintain a negative pressure at a tissue site for a given negative-pressure source. Further, the cover 125 may be vapor permeable and/or liquid impermeable, thereby allowing vapor and inhibiting liquids from exiting a sealed space between the cover 125 and the tissue site. The cover 125 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, the cover 125 may be a flexible, breathable film, membrane, or sheet having a high moisture vapor transfer rate (MVTR) of, for example, at least about 300g/m2 per 24 hours. In other embodiments, a low or no vapor transfer drape might be used. The cover 125 may comprise a range of medically suitable films having a thickness up to about 50 microns (µm). In some embodiments, an MVTR up to 5,000 grams per square meter per twenty-four hours may provide effective breathability and mechanical properties.

In some example embodiments, the cover 125 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 125 may be, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polyamide copolymers. Such materials are commercially available as, for example, Tegaderm^{®} drape, commercially available from 3M Company, Minneapolis Minnesota; polyurethane (PU) drape; polyether block polyamide copolymer (PEBAX), for example from Arkema, France; and INSPIRE 2301 and INSPIRE 2327 polyurethane films, commercially available from Expopack Advanced Coatings, Wrexham, United Kingdom. In some embodiments, the cover 125 may comprise INSPIRE 2301 having an MVTR (upright cup technique) of 2600 g/m2/24 hours and a thickness of about 30 microns, or an MVTR (inverted cup technique) of 14400 g/m2/24 hours and a thickness of about 30 microns.

An attachment device may be used to attach the cover 125 to an attachment surface, such as undamaged epidermis, a gasket, or another cover. The attachment device may take many forms. For example, an attachment device may be a medically-acceptable, pressure-sensitive adhesive configured to bond the cover 125 to epidermis around a tissue site. In some embodiments, for example, some or all of the cover 125 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The solution source 145 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

In operation, the tissue interface 120 may be placed within, over, on, or otherwise proximate to a tissue site. If the tissue site is a wound, for example, the tissue interface 120 may partially or completely fill the wound, or it may be placed over the wound. The cover 125 may be placed over the tissue interface 120 and sealed to an attachment surface near a tissue site. For example, the cover 125 may be sealed to undamaged epidermis peripheral to a tissue site. Thus, the dressing 110 can provide a sealed therapeutic environment proximate to a tissue site, substantially isolated from the external environment, and the negative-pressure source 105 can reduce pressure in the sealed therapeutic environment.

In general, exudate and other fluid flow toward lower pressure along a fluid path. Thus, the term "downstream" may refer to a location in a fluid path relatively closer to a source of negative pressure or further away from a source of positive pressure. Conversely, the term "upstream" may refer to a location further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications, such as by substituting a positive-pressure source for a negative-pressure source.

Negative pressure applied across the tissue site through the tissue interface 120 in the sealed therapeutic environment can induce macro-strain and micro-strain in the tissue site. Negative pressure can also remove exudate and other fluid from a tissue site, which can be collected in container 115.

In some embodiments, the controller 130 may receive and process data from one or more sensors, such as the first sensor 135. The controller 130 may also control the operation of one or more components of the therapy system 100 to manage the pressure delivered to the tissue interface 120. In some embodiments, controller 130 may include an input for receiving a desired target pressure and may be programmed for processing data relating to the setting and inputting of the target pressure to be applied to the tissue interface 120. In some example embodiments, the target pressure may be a fixed pressure value set by an operator as the target negative pressure desired for therapy at a tissue site and then provided as input to the controller 130. The target pressure may vary from tissue site to tissue site based on the type of tissue forming a tissue site, the type of injury or wound (if any), the medical condition of the patient, and the preference of the attending physician. After selecting a desired target pressure, the controller 130 can operate the negative-pressure source 105 in one or more control modes based on the target pressure and may receive feedback from one or more sensors to maintain the target pressure at the tissue interface 120.

In some embodiments, the controller 130 may have a continuous pressure mode, in which the negative-pressure source 105 is operated to provide a constant target negative pressure for the duration of treatment or until manually deactivated. Additionally or alternatively, the controller may have an intermittent pressure mode. For example, the controller 130 can operate the negative-pressure source 105 to cycle between a target pressure and atmospheric pressure. For example, the target pressure may be set at a value of 135 mmHg for a specified period of time (e.g., 5 min), followed by a specified period of time (e.g., 2 min) of deactivation. The cycle can be repeated by activating the negative-pressure source 105, which can form a square wave pattern between the target pressure and atmospheric pressure.

In some example embodiments, the increase in negative-pressure from ambient pressure to the target pressure may not be instantaneous. For example, the negative-pressure source 105 and the dressing 110 may have an initial rise time. The initial rise time may vary depending on the type of dressing and therapy equipment being used. For example, some therapy systems may increase negative pressure at a rate of about 20-30 mmHg/second, and other therapy systems may increase negative pressure at a rate of about 5-10 mmHg/second. If the therapy system 100 is operating in an intermittent mode, the repeating rise time may be a value substantially equal to the initial rise time.

In some example dynamic pressure control modes, the target pressure can vary with time. For example, the target pressure may vary in the form of a triangular waveform, varying between a negative pressure of 50 and 135 mmHg with a rise rate of negative pressure set at a rate of 25 mmHg/min. and a descent rate set at 25 mmHg/min. In other embodiments of the therapy system 100, the triangular waveform may vary between negative pressure of 25 and 135 mmHg with a rise rate of about 30 mmHg/min and a descent rate set at about 30 mmHg/min.

In some embodiments, the controller 130 may control or determine a variable target pressure in a dynamic pressure mode, and the variable target pressure may vary between a maximum and minimum pressure value that may be set as an input prescribed by an operator as the range of desired negative pressure. The variable target pressure may also be processed and controlled by the controller 130, which can vary the target pressure according to a predetermined waveform, such as a triangular waveform, a sine waveform, or a saw-tooth waveform. In some embodiments, the waveform may be set by an operator as the predetermined or time-varying negative pressure desired for therapy.

In some embodiments, the controller 130 may receive and process data, such as data related to instillation solution provided to the tissue interface 120. Such data may include the type of instillation solution prescribed by a clinician, the volume of fluid or solution to be instilled to a tissue site ("fill volume"), and the amount of time prescribed for leaving solution at a tissue site ("dwell time") before applying a negative pressure to the tissue site. The fill volume may be, for example, between 10 and 500 mL, and the dwell time may be between one second to 30 minutes. The controller 130 may also control the operation of one or more components of the therapy system 100 to instill solution. For example, the controller 130 may manage fluid distributed from the solution source 145 to the tissue interface 120. In some embodiments, fluid may be instilled to a tissue site by applying a negative pressure from the negative-pressure source 105 to reduce the pressure at the tissue site, drawing solution into the tissue interface 120. In some embodiments, solution may be instilled to a tissue site by applying a positive pressure from the positive-pressure source 150 to move solution from the solution source 145 to the tissue interface 120. Additionally or alternatively, the solution source 145 may be elevated to a height sufficient to allow gravity to move solution into the tissue interface 120.

The controller 130 may also control the fluid dynamics of instillation by providing a continuous flow of solution or an intermittent flow of solution. Negative pressure may be applied to provide either continuous flow or intermittent flow of solution. The application of negative pressure may be implemented to provide a continuous pressure mode of operation to achieve a continuous flow rate of instillation solution through the tissue interface 120, or it may be implemented to provide a dynamic pressure mode of operation to vary the flow rate of instillation solution through the tissue interface 120. Alternatively, the application of negative pressure may be implemented to provide an intermittent mode of operation to allow instillation solution to dwell at the tissue interface 120. In an intermittent mode, a specific fill volume and dwell time may be provided depending, for example, on the type of tissue site being treated and the type of dressing being utilized. After or during instillation of solution, negative-pressure treatment may be applied. The controller 130 may be utilized to select a mode of operation and the duration of the negative pressure treatment before commencing another instillation cycle.

Some dressings can be worn for a time period extending beyond seven days, which can be referred to as an extended wear time. Dressings for extended wear time can provide cost-savings, time-efficiencies, and less trauma to a patient during dressing changes. Some highly felted foam materials may be worn for an extended period of time without suffering tissue in-growth. However, some felted foam materials may be quite stiff. Stiffer foam materials may be hard to conform, difficult to resize, and unable to collapse under negative pressure to provide macro-strain to the tissue site. Additionally the stiff foam material may not efficiently deliver fluid and pressure to the tissue site because the manifold is unable to contract radially.

These limitations and others may be addressed by the therapy system 100, which can provide negative-pressure therapy and instillation therapy. In some embodiments, the therapy system 100 may include a tissue interface or manifold layer that can prevent tissue-ingrowth but is also flexible, collapsible, and resizable. The tissue interface or manifold layer may include a pattern enabling the manifold to contract radially in all directions, thereby increasing the effectiveness of the therapy system 100.

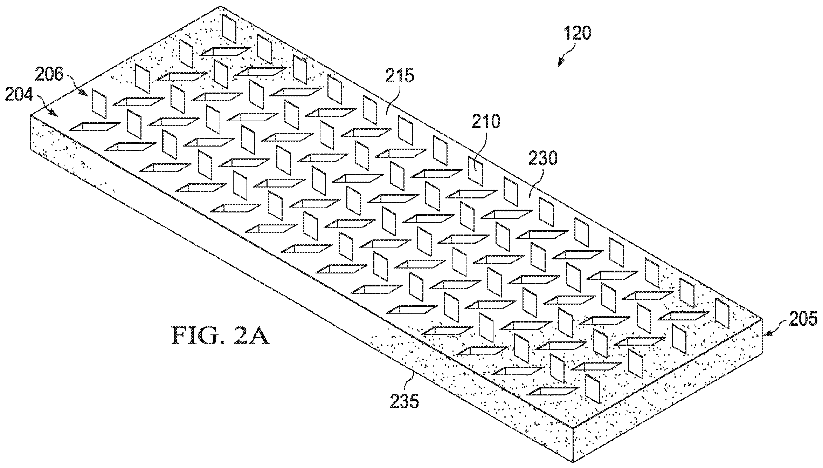

FIGURE 2A is a perspective view of an example of the tissue interface 120 of FIGURE 1, illustrating additional details that may be associated with some embodiments. Herein, various examples of the tissue interface 120 are described that may be suitable for use with the dressing 110 and the therapy system 100. Further, features or elements of the tissue interface 120 described herein may be referred to as part of the therapy system 100 or the dressing 110 without reference to the tissue interface 120.

In the example of FIGURE 2A, the tissue interface 120 may include or may be a manifold, such as a manifold layer 205. The manifold layer 205 may also provide a means for collecting or distributing fluid across the tissue interface 120 under pressure. For example, the manifold layer 205 may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed or a secondary fluid path may be provided to facilitate delivering fluid, such as from a source of instillation solution, across the tissue interface 120.

In some illustrative embodiments, the pathways of the manifold layer 205 may be interconnected to improve distribution or collection of fluids. In some illustrative embodiments, the manifold layer 205 may be a porous material having interconnected fluid pathways. Examples of suitable porous material that comprise or can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, the manifold layer 205 may additionally or alternatively include projections that form interconnected fluid pathways. For example, the manifold layer 205 may be molded to provide surface projections that define interconnected fluid pathways.

In some embodiments, the manifold layer 205 may be a foam having pore sizes that may vary according to needs of a prescribed therapy. For example, a foam having an average pore size in a range of 50-120 microns may be particularly suitable for some types of therapy. The tensile strength of the manifold layer 205 may also vary according to needs of a prescribed therapy. For example, the tensile strength of a foam may be increased for instillation of topical treatment solutions. In some embodiments, the manifold layer 205 may have a 25% compression load deflection of about 1.8 to about 2.8 pounds per square inch, and a 65% compression load deflection of about 2.2 to about 3.4 pounds per square inch. In some embodiments, the manifold layer 205 may be a foam comprised of polyols such as polyester or polyether, isocyanate such as toluene diisocyanate, and polymerization modifiers such as amines and tin compounds. In some examples, the manifold layer 205 may be a reticulated polyurethane foam such as used in GRANUFOAM^{™} Dressing or V.A.C. VERAFLO^{™} Dressing, both available from KCI of San Antonio, Texas.

In some embodiments, the manifold layer 205 may be formed by a felting process. Any porous foam suitable for felting may be used, including the example foams mentioned herein, such as GRANUFOAM^{™} Dressing. Felting comprises a thermoforming process that permanently compresses a foam to increase the density of the foam while maintaining interconnected pathways. Felting may be performed by any known methods, which may include applying heat and pressure to a porous material or foam material. Some methods may include compressing a foam blank between one or more heated platens or dies (not shown) for a specified period of time and at a specified temperature. The direction of compression may be along the thickness of the foam blank.

The period of time of compression may range from 10 minutes up to 24 hours, though the time period may be more or less depending on the specific type of porous material used. Further, in some examples, the temperature may range between 120°C to 260°C. Generally, the lower the temperature of the platen, the longer a porous material must be held in compression. After the specified time period has elapsed, the pressure and heat will form a felted structure or surface on or through the porous material or a portion of the porous material.

The felting process may alter certain properties of the original material, including pore shape and/or size, elasticity, density, and density distribution. For example, struts that define pores in the foam may be deformed during the felting process, resulting in flattened pore shapes. The deformed struts can also decrease the elasticity of the foam. The density of the foam is generally increased by felting. In some embodiments, contact with hot-press platens in the felting process can also result in a density gradient in which the density is greater at the surface and the pores size is smaller at the surface. In some embodiments, the felted structure may be comparatively smoother than any unfinished or non-felted surface or portion of the porous material. Further, the pores in the felted structure may be smaller than the pores throughout any unfinished or non-felted surface or portion of the porous material. In some examples, the felted structure may be applied to all surfaces or portions of the porous material. Further, in some examples, the felted structure may extend into or through an entire thickness of the porous material such that the all of the porous material is felted.

A felted foam may be characterized by a firmness factor, which is indicative of the compression of the foam. The firmness factor of a felted foam can be specified as the ratio of original thickness to final thickness. A compressed or felted foam may have a firmness factor greater than 1. The degree of compression may affect the physical properties of the felted foam. For example, felted foam has an increased effective density compared to a foam of the same material that is not felted. The felting process can also affect fluid-to-foam interactions. For example, as the density increases, compressibility or collapse may decrease. Therefore, foams which have different compressibility or collapse may have different firmness factors. In some example embodiments, a firmness factor can range from about 2 to about 10. For example, the firmness factor of the manifold layer 205 felted foam may be about 5 to 8 in some embodiments. There is a general linear relationship between firmness level, density, pore size (or pores per inch) and compressibility. For example, foam that is felted to a firmness factor of 5 will show a five-fold density increase and compress to about a fifth of its original thickness.

In some embodiments, the manifold layer 205 may be a closed-cell foam such as, for example, those manufactured by Zotefoams, Inc. of Walton, Kentucky, U.S.A., including the Azote, Plastazote, Evazote, Supazote, and Zotek grades. Such closed-cell foams may be manufactured by extruding polymer sheets or blocks and crosslinking through high energy radiation. Suitable polymers may include low and high density polyolefins and copolymers with vinyl acetate, fluoropolymers, polyamides, and PEBAX. The polymer sheets may then be softened under heat and exposed to high pressure nitrogen gas which dissolves in the polymer. After cooling, the polymer sheets may be heated again and exposed to subatmospheric pressure, resulting in expansion and formation of the closed-cell foam. In some embodiments, the closed-cell foam may be thermoformed. Thermoforming the foam involves embossing patterns and structures, such as channels, into the surfaces of the foam in order to translate what would otherwise be non-manifolding material into a structure that will manifold pressure and fluid along the channels.

In some embodiments, one or more suitable foam blanks may be used for forming the manifold layer 205. The foam blanks may be open cell foam, felted foam, closed cell foam, or another foam as described herein. However, the properties of any of these foam blanks may have about 200 to about 400 pores per inch on average, a density of about 6.5 to about 12.8 lb/ft3, a free volume of about 90% or more, an average pore size in a range of about 50 to about 120 microns, and/or a 25% compression load deflection of at least 1.5 pounds per square inch. In some embodiments, the foam blank(s) may have a thickness greater than 8 millimeters, for example 8-16 millimeters.

As further shown in FIGURE 2A, the manifold layer 205 may comprise one or more holes or contraction apertures 210 extending through a first side 230, a second side 235 opposite the first side 230, and a thickness of the manifold layer 205. The thickness of the manifold layer 205 may be between about 8-16 millimeters. The plurality of contraction apertures 210 may be distributed uniformly or randomly across the manifold layer 205. In some embodiments, the plurality of contraction apertures 210 may be positioned in at least a first row 204 and a second row 206 that is offset or staggered from the first row 204. The plurality of contraction apertures 210 extending through the manifold layer 205 may form a plurality of walls 215 extending through the manifold layer 205. The contraction apertures 210 may be configured to contract radially in all directions in response to the application of negative pressure to the tissue interface 120 such that there is radial mechanical deformation at the tissue site.

In some embodiments, the contraction apertures 210 may be formed during molding of the manifold layer 205. In other embodiments, the contraction apertures 210 may be formed by cutting, melting, or vaporizing the manifold layer 205. For example, the contraction apertures 210 may be formed in the manifold layer 205 by laser cutting the felted foam of the manifold layer 205.

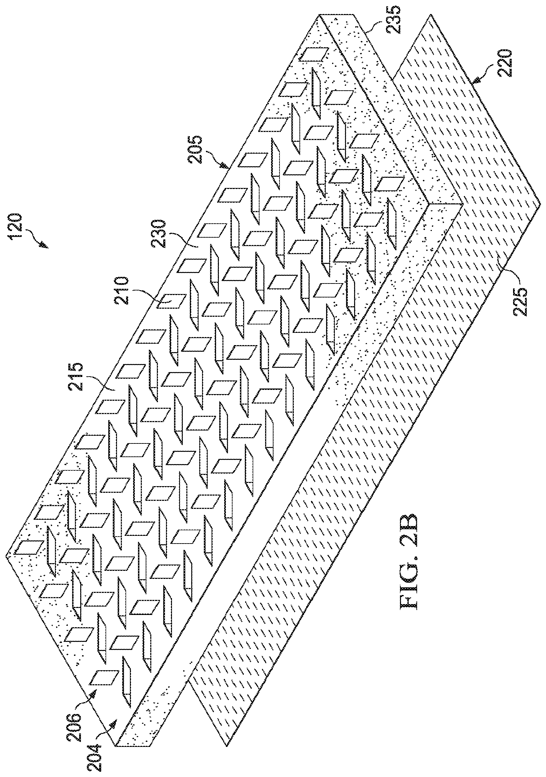

FIGURE 2B is an exploded view of another example of the tissue interface 120 of FIGURE 1, illustrating additional details that may be associated with some embodiments. In some embodiments, the tissue interface 120 may comprise the manifold layer 205 and an optional contact layer, which in some embodiments may be a non-adherent drape or a substantially fluid impermeable layer, such as a film layer 220. In some embodiments, the film layer 220 may optionally be disposed adjacent to the manifold layer 205. For example, the film layer 220 and the manifold layer 205 may be stacked so that the film layer 220 is in contact with the manifold layer 205. The film layer 220 may also be heat-bonded or adhered to the manifold layer 205 in some embodiments, for example, using hot melt adhesive. In some embodiments, the film layer 220 may optionally include a low-tack adhesive, which can be configured to hold the tissue interface 120 in place while the cover 125 is applied. The low-tack adhesive may be continuously coated on the film layer 220 or applied in a pattern. In some embodiments, the film layer 220 may be configured to be positioned between the manifold layer 205 and a tissue site. In some embodiments, the film layer 220 may be configured to be positioned in direct contact with the tissue site. The film layer 220 can provide additional protection to the epidermis from irritation that could be caused by expansion, contraction, or other movement of the manifold layer 205. The film layer 220 can also reduce tissue in-growth into the manifold layer 205.

The film layer 220 may include a means for controlling or managing fluid flow. In some embodiments, the film layer 220 may be a fluid control layer comprising a liquid-impermeable, elastomeric material. For example, the film layer 220 may be a polymer film, such as a polyurethane film. In some embodiments, the film layer 220 may be the same material as the cover 125. The film layer 220 may also have a smooth or matte surface texture in some embodiments. A glossy or shiny finish finer or equal to a grade B3 according to the SPI (Society of the Plastics Industry) standards may be particularly advantageous for some applications. In some embodiments, variations in surface height may be limited to acceptable tolerances. For example, the surface of the film layer 220 may have a substantially flat surface, with height variations limited to 0.2 millimeters over a centimeter.

In some embodiments, the film layer 220 may be hydrophobic. The hydrophobicity of the film layer 220 may vary, but may have a contact angle with water of at least ninety degrees in some embodiments. In some embodiments the film layer 220 may have a contact angle with water of no more than 150 degrees. For example, in some embodiments, the contact angle of the film layer 220 may be in a range of at least 90 degrees to about 120 degrees, or in a range of at least 120 degrees to 150 degrees. Water contact angles can be measured using any standard apparatus. Although manual goniometers can be used to visually approximate contact angles, contact angle measuring instruments can often include an integrated system involving a level stage, liquid dropper such as a syringe, camera, and software designed to calculate contact angles more accurately and precisely, among other things. Non-limiting examples of such integrated systems may include the FTÅ125, FTÅ200, FTÅ2000, and FTÅ4000 systems, all commercially available from First Ten Angstroms, Inc., of Portsmouth, VA, and the DTA25, DTA30, and DTA100 systems, all commercially available from Kruss GmbH of Hamburg, Germany. Unless otherwise specified, water contact angles herein are measured using deionized and distilled water on a level sample surface for a sessile drop added from a height of no more than 5 cm in air at 20-25°C and 20-50% relative humidity. Contact angles herein represent averages of 5-9 measured values, discarding both the highest and lowest measured values. The hydrophobicity of the film layer 220 may be further enhanced with a hydrophobic coating of other materials, such as silicones and fluorocarbons, either as coated from a liquid, or plasma coated.

The film layer 220 may also be suitable for welding to other layers, including the manifold layer 205. For example, the film layer 220 may be adapted for welding to polyurethane foams using heat, radio frequency (RF) welding, or other methods to generate heat such as ultrasonic welding. RF welding may be particularly suitable for more polar materials, such as polyurethane, polyamides, polyesters and acrylates. Sacrificial polar interfaces may be used to facilitate RF welding of less polar film materials, such as polyethylene. In some embodiments, the film layer 220 may be flame laminated to the manifold layer 205.

The area density of the film layer 220 may vary according to a prescribed therapy or application. In some embodiments, an area density of less than 40 grams per square meter may be suitable, and an area density of about 20-30 grams per square meter may be particularly advantageous for some applications.

In some embodiments, for example, the film layer 220 may be a hydrophobic polymer, such as a polyethylene film. The simple and inert structure of polyethylene can provide a surface that interacts little, if any, with biological tissues and fluids, providing a surface that may encourage the free flow of liquids and low adherence, which can be particularly advantageous for many applications. Other suitable polymeric films include polyurethanes, acrylics, polyolefin (such as cyclic olefin copolymers), polyacetates, polyamides, polyesters, copolyesters, PEBAX block copolymers, thermoplastic elastomers, thermoplastic vulcanizates, polyethers, polyvinyl alcohols, polypropylene, polymethylpentene, polycarbonate, styreneics, silicones, fluoropolymers, and acetates. A thickness between 20 microns and 100 microns may be suitable for many applications. Films may be clear, colored, or printed. More polar films suitable for laminating to a polyethylene film include polyamide, co-polyesters, ionomers, and acrylics. To aid in the bond between a polyethylene and polar film, tie layers may be used, such as ethylene vinyl acetate, or modified polyurethanes. An ethyl methyl acrylate (EMA) film may also have suitable hydrophobic and welding properties for some configurations.

The film layer 220 may have one or more passages, which can be distributed uniformly or randomly across the film layer 220. In some embodiments, the passages may be bidirectional and pressure-responsive. For example, each of the passages generally may be an elastic passage that is normally unstrained to substantially reduce liquid flow, and can expand or open in response to a pressure gradient and/or in response to the contraction of the manifold layer 205. As illustrated in the example of FIGURE 2B, the passages may be a plurality of perforations 225 disposed through opposing surfaces of the film layer 220. The plurality of perforations 225 may be formed by removing material from the film layer 220. For example, the plurality of perforations 225 may be formed by cutting through the film layer 220. In the absence of a pressure gradient across the plurality of perforations 225, the plurality of perforations 225 may be sufficiently small to form a seal or fluid restriction, which can substantially reduce or prevent liquid flow. Additionally, or alternatively, one or more of the passages may be or may function as an elastomeric valve that is normally closed when unstrained to substantially prevent liquid flow, and can open in response to a pressure gradient and/or in response to the contraction of the manifold layer 205. In some examples, the passages may be fenestrations in the film layer 220. Generally, fenestrations are a species of perforation, and may also be formed by removing material from the film layer 220. The amount of material removed and the resulting dimensions of the fenestrations may be up to an order of magnitude less than perforations.

In some embodiments, the plurality of perforations 225 may be formed as slots (or fenestrations formed as slits) in the film layer 220. In some examples, the plurality of perforations 225 may be linear slots having a length less than 4 millimeters and a width less than 1 millimeter. The length may be at least 2 millimeters, and the width may be at least 0.4 millimeters in some embodiments. A length of about 3 millimeters and a width of about 0.8 millimeters may be particularly suitable for many applications, and a tolerance of about 0.1 millimeter may also be acceptable. Such dimensions and tolerances may be achieved with a laser cutter, for example. Slots of such configurations may function as imperfect elastomeric valves that can substantially reduce liquid flow in a normally closed or resting state. For example, such slots may form a flow restriction without being completely closed or sealed. The slots can expand or open wider in response to a pressure gradient and/or in response to the contraction of the manifold layer 205 to allow increased liquid flow.

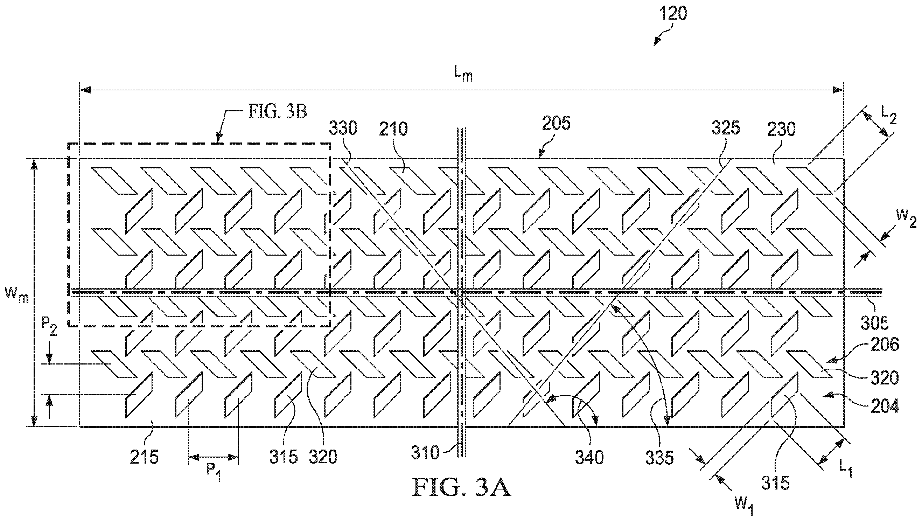

FIGURE 3A is a top view of the tissue interface of FIGURE 2A, illustrating additional details that may be associated with some embodiments. As shown in FIGURE 3A, in some embodiments, the tissue interface 120 may have a first orientation line 305 and a second orientation line 310 that is perpendicular to the first orientation line 305. In some embodiments, the first orientation line 305 may be parallel to a length LM of the tissue interface 120 and the manifold layer 205, and the second orientation line 310 may be parallel to a width WM of the tissue interface 120 and the manifold layer 205. Generally, the first orientation line 305 and the second orientation line 310 aid in the description of the tissue interface 120.

In some embodiments, the manifold layer 205 may be configured to contract radially in all directions in a plane under the application of negative pressure. For example, the length LM of the manifold layer 205 and the width WM of the manifold layer 205 may lie in a contraction plane where the manifold layer 205 is configured to radially contract in all directions in the contraction plane or a planar area defined by the first side 230 and/or the second side 235 of the manifold layer 205. For example, if the tissue interface 120 is not subjected to negative pressure, the length LM may be a nominal or relaxed length, and the width WM may be a nominal or relaxed width. In some embodiments, the length LM may be greater than the width WM, such that the width WM, may be less than the length LM. For example, the tissue interface 120 may have an elongate shape. In some embodiments, for example, the tissue interface 120 may have a length LM to width WM ratio of at least 4:1. In some embodiments, the length LM may be equal to the width WM. In some embodiments, the length LM may be less than the width WM.

Although the manifold layer 205 is shown as having an elongate rectangular shape, the manifold layer 205 may have other shapes. For example, the manifold layer 205 may have a stadium, diamond, square, oval or circular shape. In some embodiments, the shape of the manifold layer 205 may be selected to accommodate the type of tissue site being treated. For example, the manifold layer 205 may have an oval or circular shape to accommodate an oval or circular tissue site. In other example embodiments, the manifold layer 205 may be resized and formed into any desirable shape by a clinician prior to placement at the tissue site.

As further illustrated in FIGURE 3A, the contraction apertures 210 may include a first plurality of contraction apertures 315 in the first row 204 and a second plurality of contraction apertures 320 in the second row 206. Additional rows of holes may be provided, as shown in the non-limiting embodiment of FIGURE 3A. Each of the first plurality of contraction apertures 315 and the second plurality of contraction apertures 320 may be configured to have an elongate shape that extends through the first side 230, the second side 235, and the thickness of the manifold layer 205.

In some embodiments, each of the first plurality of contraction apertures 315 and the second plurality of contraction apertures 320 may have a length that is longer than and perpendicular to a hole width. In some embodiments, each of the first plurality of contraction apertures 315 and the second plurality of contraction apertures 320 may have a shape configured to be a parallelogram. In some embodiments where the contraction apertures 210 are parallelogram-shaped, each of the first plurality of contraction apertures 315 may have a length L1 and a width W1 perpendicular to the length L1, and each of the second plurality of contraction apertures 320 may have a length L2 and a width W2 perpendicular to the length L2. In the example of FIGURE 3A, L1 and L2 may be about 10-17 millimeters, and W1 and W2 may be about 3-8 millimeters. In some embodiments, the length L1 may be equal to the length L2. In some embodiments, the width W1 may be equal to the width W2. In other embodiments, L1 and L2 may be substantially equal, and W1 and W2 may be substantially equal, within acceptable manufacturing tolerances.

The first plurality of contraction apertures 315 and the second plurality of contraction apertures 320 may be distributed across the manifold layer 205 in one or more rows in one direction or in different directions. In some embodiments, the rows of the first plurality of contraction apertures 315 and the second plurality of contraction apertures 320 may be offset or staggered. In some embodiments, the length L1 of one or more of the first plurality of contraction apertures 315 in the first row 204 may point toward the length L2 of one or more of the second plurality of contraction apertures 320 in the second row 206. In some embodiments, the length L2 of one or more of the second plurality of contraction apertures 320 in the second row 206 may point toward the length L1 of one or more of the first plurality of contraction apertures 315 in the first row 204.

In some embodiments, the first plurality of contraction apertures 315 in the first row 204 may overlap with the second plurality of contraction apertures 320 in the second row 206. For example, at least a portion of the first plurality of contraction apertures 315 in the first row 204 would also be positioned or extend into the second row 206 and at least a portion of the second plurality of contraction apertures 320 in the second row 206 would also be positioned or extend into the first row 204. In other embodiments, the first plurality of contraction apertures 315 in the first row 204 may not overlap with the second plurality of contraction apertures 320 in the second row 206. For example, the first plurality of contraction apertures 315 may be positioned entirely in the first row 204 separate from the second plurality of contraction apertures 320 positioned entirely in the second row 206.

In some embodiments, the length L1 of one or more of the first plurality of contraction apertures 315 in the first row 204 may be positioned at an angle relative to the length L2 of one or more of the second plurality of contraction apertures 320 in the second row 206. In some embodiments, the length L1 of one or more of the first plurality of contraction apertures 315 in the first row 204 may be angled toward the length L2 of one or more of the second plurality of contraction apertures 320 in the second row 206. In some embodiments, the length L1 of one or more of the first plurality of contraction apertures 315 in the first row 204 may form an angle of about 90 degrees relative to the length L2 of one or more of the second plurality of contraction apertures 320 in the second row 206. In some embodiments, the length L1 of one or more of the first plurality of contraction apertures 315 in the first row 204 may form an angle of about 45 degrees relative to the width W2 of one or more of the second plurality of contraction apertures 320 in the second row 206.

In some embodiments, the first plurality of contraction apertures 315 in the first row 204 and the second plurality of contraction apertures 320 in the second row 206 may extend along the length LM of the manifold layer 205. In some embodiments, the length L1 of the first plurality of contraction apertures 315 and the length L2 of the second plurality of contraction apertures 320 may be positioned at an angle relative to the length LM of the manifold layer 205. For example, each of the first plurality of contraction apertures 315 may have a first long axis, such as a first reference line 325, which may be parallel to the length L1 of the first plurality of contraction apertures 315. Each of the second plurality of contraction apertures 320 may have a second long axis, such as a second reference line 330, which may be parallel to the length L2 of the second plurality of contraction apertures 320. In some embodiments, one or both of the first reference line 325 and the second reference line 330 may be defined relative to the length LM. For example, the first reference line 325 may form a first angle 335 relative to the length LM and the second reference line 330 may form a second angle 340 relative to the length LM. In some embodiments, the first angle 335 may be about 45° relative to the length LM and the second angle 340 may be about 135° relative to the length LM. In some embodiments, the first angle may about 135° relative to the length LM and the second angle may be about 45° relative to the length LM.

The pattern of contraction apertures 210 may be characterized by a pitch, which indicates the spacing between corresponding points on contraction apertures 210 within a pattern. In example embodiments, pitch may indicate the spacing between the centroids of contraction apertures 210 within the pattern. Some patterns may be characterized by a single pitch value, while others may be characterized by at least two pitch values. For example, if the spacing between centroids of the contraction apertures 210 is the same in all orientations, the pitch may be characterized by a single value indicating the spacing between centroids in adjacent rows. In some embodiments, a pattern comprising the first plurality of contraction apertures 315 and the second plurality of contraction apertures 320 may be characterized by two pitch values, P1 and P2. P1 may be the spacing between the centroid of one or more of the first plurality of contraction apertures 315 and another of the first plurality of contraction apertures 315 in the first row 204 along the first orientation line 305. P2 may be the spacing between the centroid of one or more of the first plurality of contraction apertures 315 in the first row 204 and one or more of the second plurality of contraction apertures 320 in the second row 206 perpendicular to the first orientation line 305. In some embodiments, P1 may be about 18-22 millimeters and P2 may be about 10-15 millimeters.

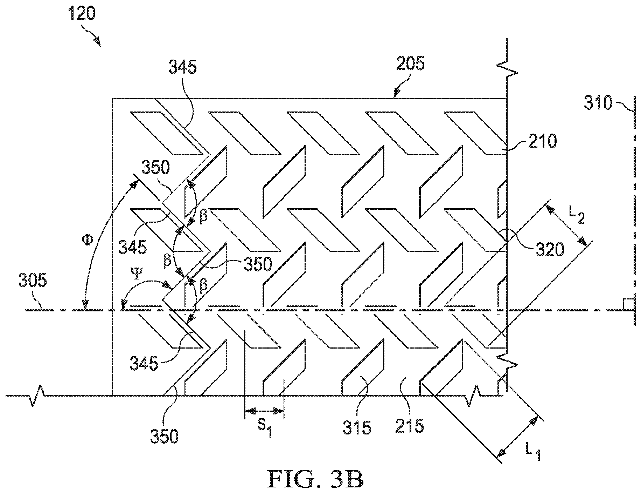

FIGURE 3B is a detail view of the manifold layer 205 taken at reference FIGURE 3B in FIGURE 3A. In some embodiments, the rows may be offset or staggered. The stagger may be characterized by an orientation of corresponding points in successive rows relative to an edge or other reference line associated with the manifold layer 205. In some embodiments, the rows of the first plurality of contraction apertures 315 may be staggered with the rows of the second plurality of contraction apertures 320. In some embodiments, the stagger may be characterized by a distance or a stagger value S1, where S1 may be the spacing between the centroid of one or more of the first plurality of contraction apertures 315 in the first row 204 and one or more of the second plurality of contraction apertures 320 in the second row 206 in a direction parallel to the to the first orientation line 305.

In some embodiments, the plurality of walls 215 may form a web including a plurality of alternating first wall portions 345 and second wall portions 350. The first wall portions 345 may be oriented at an angle Φ with respect to the first orientation line 305. In the example of FIGURE 3B, the first wall portions 345 may be about 45° with respect to the first orientation line 305. The second wall portions 350 may be oriented at an angle Ψ with respect to the first orientation line 305. In the example of FIGURE 3B, the second wall portions 350 may be about 135° with respect to the first orientation line 305. In some embodiments, the first wall portions 345 may be parallel to the length L1 of the first plurality of contraction apertures 315, and the second wall portions 350 may be parallel to the length L2 of the second plurality of contraction apertures 320. The first wall portions 345 and the second wall portions 350 may have an angle β of about 90° between each wall portion.

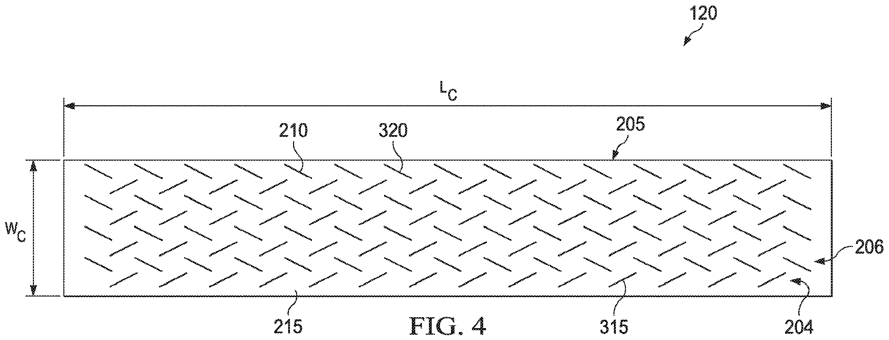

FIGURE 4 is a top view of an example of the manifold layer 205 of FIGURE 2A in a contracted position, illustrating additional details that may be associated with some embodiments. As shown in FIGURE 4, if negative pressure is applied to the tissue interface 120, the plurality of contraction apertures 210 of the manifold layer 205 may collapse or contract from a relaxed position to a contracted position. In some embodiments, the manifold layer 205 may be configured to contract to the contracted position when exposed to a reduced pressure of about 125 mm Hg. Contraction may occur in all directions as the contraction apertures 210 become smaller under the compressive force of negative pressure and apposition forces may be generated. The contraction apertures 210 aid in the delivery of negative pressure and the removal of fluids even when the contraction apertures 210 are collapsed. When instillation therapy is used, the contraction apertures 210 may also aid in the delivery of fluids even when contracted. Additionally, if negative pressure is applied to the tissue interface 120, the manifold layer 205 may collapse or contract to a contracted length and a contracted width, wherein the contracted length and the contracted width is less than a relaxed length and a relaxed width in the absence of negative pressure. Under an applied negative pressure, the tissue interface 120 may collapse or contract to a contracted length LC and a contracted width WC that is less than the nominal or relaxed length LM and the nominal or relaxed width WM of the tissue interface 120. As the tissue interface 120 contracts, the contraction may be applied to the tissue site.

In some embodiments, the manifold layer 205 may comprise a first surface area when in the relaxed position and a second surface area when in the contracted position. In a contracted position, such as shown in FIGURE 4, the second surface area may be between about 20-35 percent less than the first surface area. In some embodiments, the second surface area may be at least 20 percent less than the first surface area.

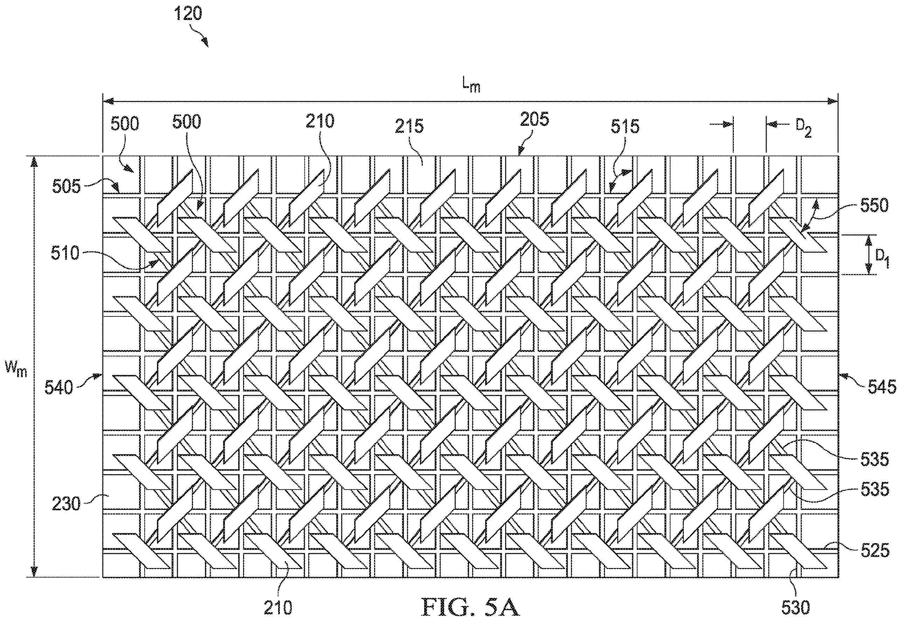

FIGURE 5A is a top view of another example of a manifold layer 205 that can be associated with some embodiments of the tissue interface 120 of FIGURES 2A and 2B. In some embodiments, the manifold layer 205 may comprise at least one surface channel 500 having a channel length extending along at least one manifold surface or side of the manifold layer 205. For example, the channel length of the at least one surface channel 500 may extend along one or both of the first side 230 or the second side 235 of the manifold layer 205. In some embodiments, the at least one surface channel 500 may be a plurality of surface channels 500. In some embodiments, the plurality of surface channels 500 may also intersect the plurality of contraction apertures 210. The plurality of surface channels 500 may also intersect one another at an intersection angle 515 to form one or more grid patterns as described herein.

For example, the plurality of surface channels 500 may intersect to form a first grid 505 and a second grid 510. A first plurality of surface channels 525 and a second plurality of surface channels 530 of the plurality of surface channels 500 may intersect to form the first grid 505. In some embodiments, the first plurality of surface channels 525 and the second plurality of surface channels 530 of the first grid 505 may intersect to form the intersection angle 515. The intersection angle 515 may be about 90 degrees and form a square grid pattern. In some embodiments, the plurality of surface channels 500 may further comprise a third plurality of surface channels 535. The third plurality of surface channels 535 may intersect the first plurality of surface channels 525 and the second plurality of surface channels 530 to form the second grid 510. In some embodiments, the third plurality of surface channels 535 may intersect the first plurality of surface channels 525 and the second plurality of surface channels 530 at the same point where the first plurality of surface channels 525 and the second plurality of surface channels 530 intersect. In some embodiments, the third plurality of surface channels 535 may intersect the first grid 505 to form the second grid 510. The third plurality of surface channels 535 may intersect the first grid 505 at a grid angle 550. In some embodiments, the grid angle 550 may be about 45 degrees.

In some embodiments, the plurality of surface channels 500 may be spaced apart by a separation distance. For example, each of the first plurality of surface channels 525 may extend from a first end 540 of the manifold layer 205 to a second end 545 opposite the first end 540. In some embodiments, each of the first plurality of surface channels 525 may be spaced apart along the width WM of the manifold layer 205 by a distance D 1. The second plurality of surface channels 530 may be perpendicular to the first plurality of surface channels 525. In some embodiments, each of the second plurality of surface channels 530 may be spaced apart along the length LM of the manifold layer 205 by a distance D2. In the example of FIGURE 5A, D1 and D2 may be between about 8 millimeters to about 12 millimeters.

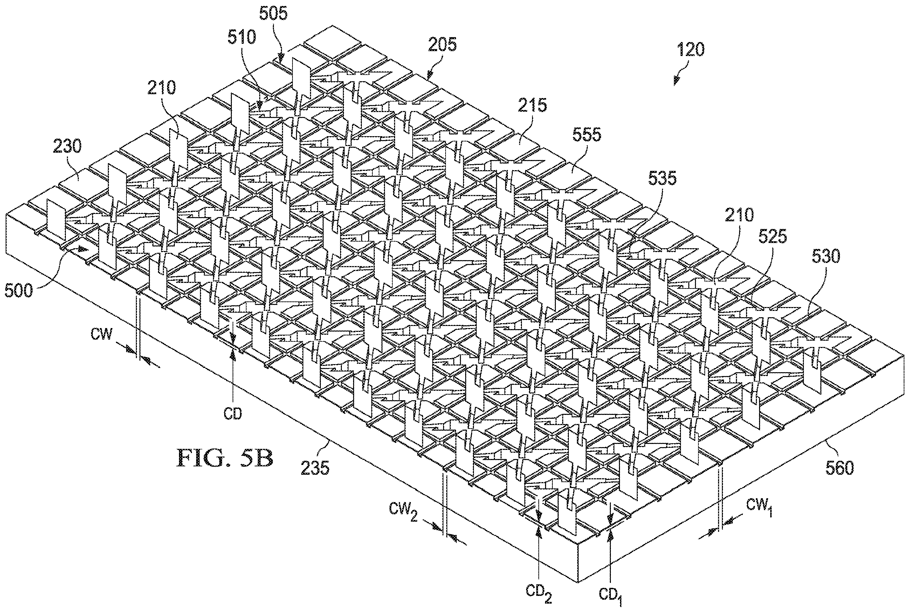

FIGURE 5B is a perspective view of the manifold layer 205 of FIGURE 5A, illustrating additional details that may be associated with some embodiments. In some embodiments, the plurality of surface channels 500 may comprise a channel depth CD and a channel width CW perpendicular to the channel depth CD. The channel depth CD may extend into the thickness of the manifold layer 205 from a manifold surface, such as one or both of a first manifold surface 555 or a second manifold surface 560 opposite the first manifold surface 555. Although not shown in FIGURE 5B, the plurality of surface channels 500 may be positioned on the second manifold surface 560 in addition to or in lieu of the plurality of surface channels 500 shown on the first manifold surface 555. In some embodiments, the channel depth CD may be between about 1 millimeter to about 8 millimeters and the channel width CW may be between about 0.5 millimeters to about 2 millimeters.

In some embodiments, the first plurality of surface channels 525 and the second plurality of surface channels 530 of the plurality of surface channels 500 may each have a first channel depth CD1 and a second channel depth CD2, respectively. In some embodiments, the second channel depth CD2 is greater than the first channel depth CD1 (CD2 > CD1). In some embodiments, the third plurality of surface channels 535 forming the second grid 510 may have a third channel depth CD3 (not shown). In some embodiments, the first channel depth CD1 and the second channel depth CD2 may be equal so that the first plurality of surface channels 525 and the second plurality of surface channels 530 that form the first grid 505 have the same depth (CD2 = CD1). In some embodiments, the third channel depth CD3 is greater than both first channel depth CD1 and the second channel depth CD2 so that the depth of the second grid 510 is greater than the depth of the first grid 505 (CD3 > [CD2 = CD1]).

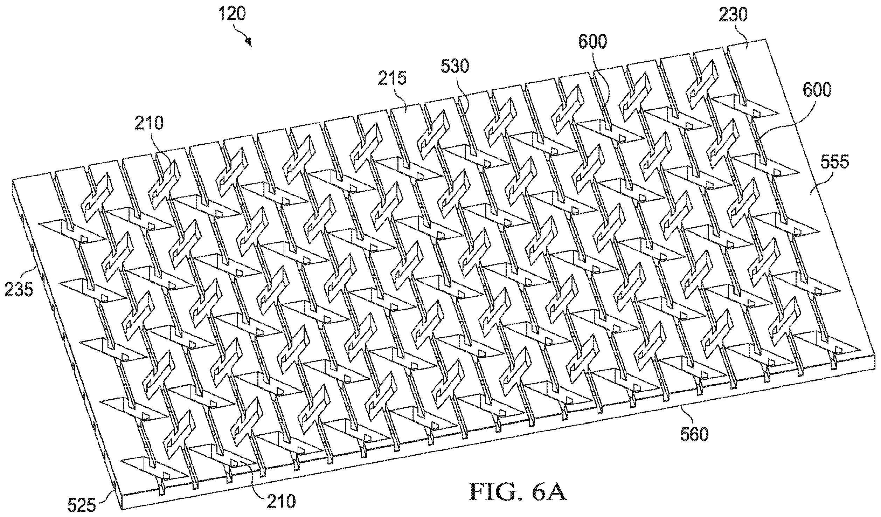

FIGURE 6A is a perspective view of another example of a manifold layer 205 that can be associated with some embodiments of the tissue interface 120 of FIGURES 2A and 2B. In some embodiments, the plurality of surface channels 500 may extend along the first manifold surface 555 of the manifold layer 205. In some embodiments, the plurality of surface channels 500 may extend along both the first manifold surface 555 and the second manifold surface 560. For example, the second plurality of surface channels 530 may extend along the first manifold surface 555 and the first plurality of surface channels 525 may extend along the second manifold surface 560.

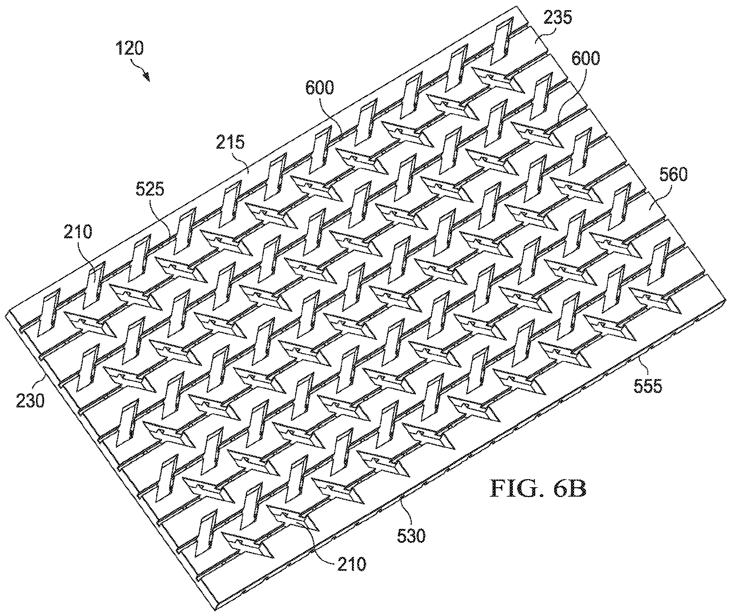

FIGURE 6B is a perspective view of the second side 235 of the tissue interface 120 of FIGURE 6A, illustrating additional features that may be associated with some embodiments. In some embodiments, the first plurality of surface channels 525 may extend along the second manifold surface 560 at a 90 degree angle relative to the second plurality of surface channels 530 extending along the first manifold surface 555. Referring to FIGURES 6A and 6B, the second channel depth CD2 of the second plurality of surface channels 530 may extend from the first manifold surface 555 toward the second manifold surface 560, and the first channel depth CD1 of the first plurality of surface channels 525 may extend from the second manifold surface 560 toward the first manifold surface 555. In some embodiments, the first channel depth CD1 may intersect with the second channel depth CD2 to form a channel aperture 600 that extends through the first manifold surface 555, the second manifold surface 560, and the thickness of the manifold layer 205.

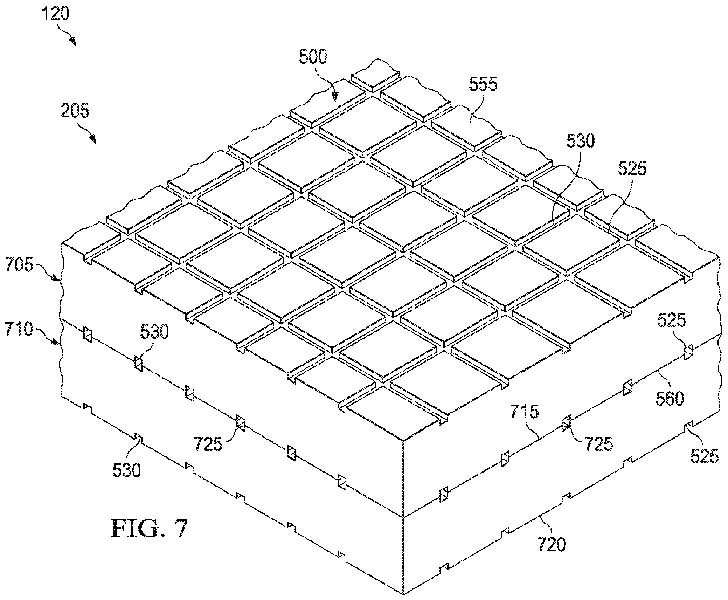

FIGURE 7 is a perspective, side view of another example of a manifold layer 205 that can be associated with some embodiments of the tissue interface 120 of FIGURE 1. In some embodiments, the manifold layer 205 may be formed by two or more layers. For example, the manifold layer 205 may comprise a first manifold layer 705 and a second manifold layer 710. Similar to embodiments of the manifold layer 205, the first manifold layer 705 may comprise the first manifold surface 555 and the second manifold surface 560. The second manifold layer 710 may comprise a third manifold surface 715 and a fourth manifold surface 720. In some embodiments, the first manifold surface 555, the second manifold surface 560, the third manifold surface 715, and the fourth manifold surface 720 may each comprise the plurality of surface channels 500 In some embodiments, the first manifold surface 555, the second manifold surface 560, the third manifold surface 715, and the fourth manifold surface 720 may each comprise the first plurality of surface channels 525 and the second plurality of surface channels 530.

In some embodiments, the first manifold layer 705 and the second manifold layer 710 may be bonded together to form the manifold layer 205. In some embodiments, the second manifold surface 560 of the first manifold layer 705 may be bonded to the third manifold surface 715 of the second manifold layer 710. In some embodiments, the first plurality of surface channels 525 on the second manifold surface 560 may be aligned with the first plurality of surface channels 525 on the third manifold surface 715, and the second plurality of surface channels 530 on the second manifold surface 560 may be aligned with the second plurality of surface channels 530 on the third manifold surface 715 to form at least one section channel 725 extending into the thickness of the manifold layer 205 substantially parallel to the first manifold surface 555, the second manifold surface 560, the third manifold surface 715, and the fourth manifold surface 720. In some embodiments, the at least one section channel may be a plurality of section channels 725. In other embodiments, the manifold layer 205 may be formed using a single layer, such as the manifold layer 205, and perforating the manifold layer 205 along the length and width of the manifold layer 205 to form the plurality of section channels 725.

In other embodiments, the manifold layer 205 may be a laminate of different densities of closed-cell foam. In such an embodiments, the manifold layer 205 may feel more flexible. In other embodiments, the laminate forming the manifold layer 205 may comprise three layers, such as two outer layers and an inner layer. The inner layer may be sandwiched between the outer layers. The outer layers may be stiffer than the inner layer, and the inner layer may be softer and more likely to conform. In still other embodiments, the manifold layer 205 may be perforated along its length and width through the closed-cell foam to provide lateral manifolding channels, such as the plurality of section channels 725. The plurality of section channels 725 may be formed by bonding at least two laminated layers where channels are thermoformed onto the surfaces of the laminated layers. The bonding process does not seal the channels closed. As a result, each thinner layer of closed-cell foam would be embossed on each side and then bonded to form a multi-orientation manifolding structure. Suitable adhesives for bonding include hot melts. The hot melts may be pattern coated onto the surfaces so as not to block the channels. In some embodiments, an adhesive may be sprayed onto the surfaces of the manifold layer 205. A fine mist of adhesive is unlikely to block the channels if the channels are about 2-3 millimeters deep. Additionally or alternatively, solvent borne adhesives may be used, such as acrylic or reactive polyurethanes. A form of heat lamination may also be used, where surface localized heat is applied to the foam layers to soften or tackify the bonding surfaces before pressing the foam layers together. Two-part reactive adhesives may also be used where one surface is coated with a first adhesive and another surface is coated with a second adhesive. The surfaces comprising the first adhesive and the second adhesive are then brought together to form the bond.

In other embodiments, one or both of the outer surfaces of the manifold layer 205 may include a texture. For example, one or both of the first manifold surface 555 and the fourth manifold surface 720 may include a texture. The texture may allow areas of the manifold layer 205 not containing channels to form manifolding areas. In some embodiments, the texture may comprise a random pattern of peaks, such as a tough Standex finish; a leather-effect patterns; a patterns of pyramids; a patterns of triangles; or a pattern of other shapes. Additionally or alternatively, one or both of the outer surfaces of the manifold layer 205 may be coated with hydrophilic or hydrophobic materials, for example by a plasma coating, to modify the fluid distribution properties of the manifold layer 205. In some embodiments, the channels of the manifold layer 205 may be specifically coated with hydrophilic or hydrophobic materials.

In still other embodiments, all or some of the layers forming the manifold layer 205 may be different colors. The differing colors may improve visualization of fluids, such as bleeding. A full range of colors may be used to form the manifold layer 205.

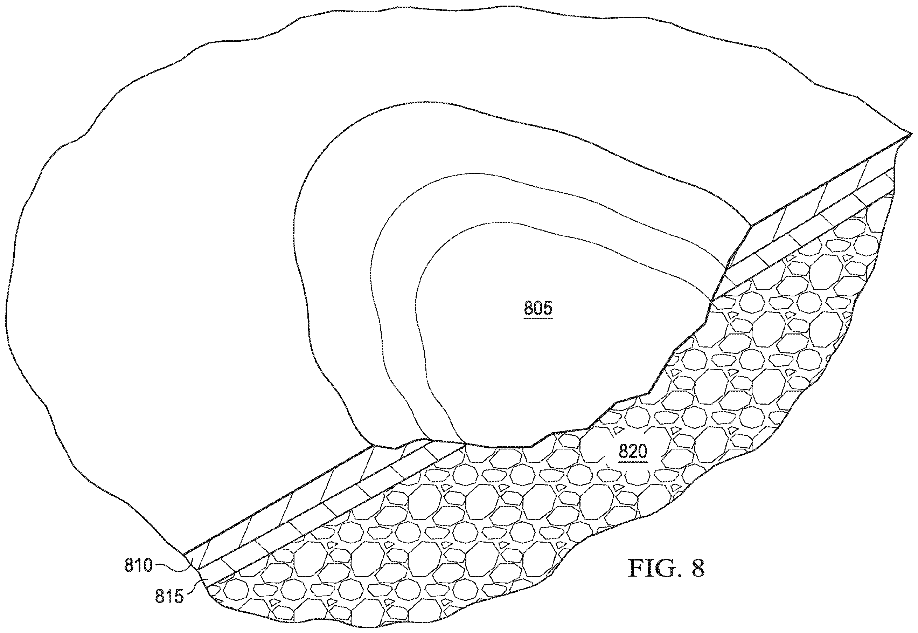

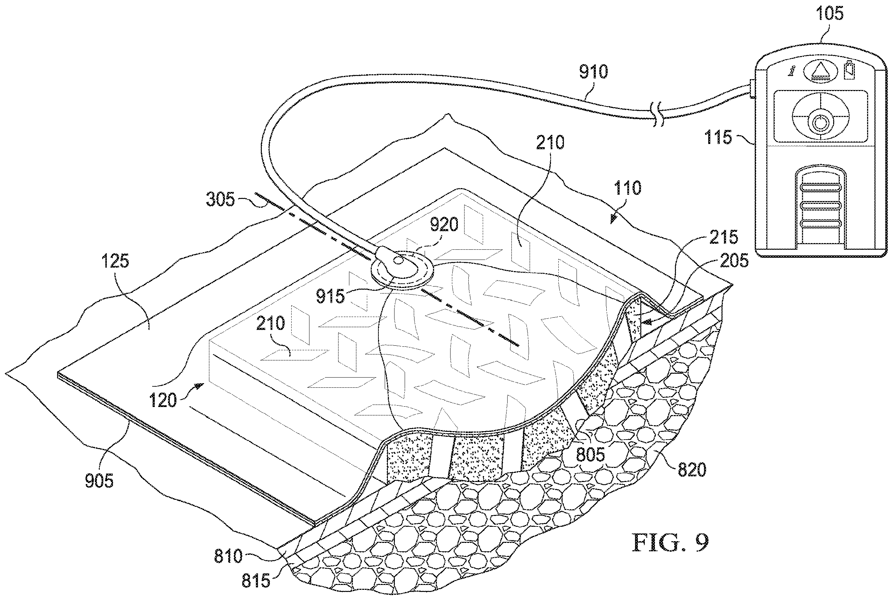

Referring primarily to FIGURES 8 and 9, presented is an illustrative embodiment of a portion of the therapy system 100. FIGURES 8 and 9 depict the therapy system 100 assembled in stages at a tissue site 805, which may be a wound. In some embodiments, the tissue site 805 may be a deep wound. In some embodiments, the tissue site 805 may include a portion through an epidermis 810, dermis 815, and subcutaneous tissue 820. Referring now to FIGURE 9, the dressing 110 may be disposed within the tissue site 805. The geometry and dimensions of the tissue interface 120, the cover 125, or both may vary to suit a particular application or anatomy. For example, the dressing 110 may be cut to size for a specific region or anatomical area. The dressing 110 may be cut without losing pieces of the tissue interface 120 and without separation of the tissue interface 120. In other embodiments, the dressing 110 may be placed proximate to the tissue site 805.

The tissue interface 120 can be placed in, over, on, or otherwise proximate to the tissue site 805. The manifold layer 205 may be cut to size, folded, and rolled into the tissue site 805. In some embodiments, the cover 125 may be placed over the manifold layer 205. The cover 125 may be configured to create a sealed space containing the manifold layer 205 at the tissue site. The negative-pressure source 105 may be configured to be positioned in fluid communication with the sealed space and the manifold layer 205 through the cover 125.

In some examples, the dressing 110 may include one or more attachment devices. In some embodiments, one or more of the attachment devices may include an adhesive 905. In some examples the adhesive 905 may be, for example, a medically-acceptable, pressure-sensitive adhesive that extends about a periphery, a portion, or an entire surface of each of the cover 125. In some embodiments, for example, the adhesive 905 may be an acrylic adhesive having a coating weight between 25-65 grams per square meter (g.s.m.). Thicker adhesives, or combinations of adhesives, may be applied in some embodiments to improve the seal and reduce leaks. In some embodiments, such a layer of the adhesive 905 may be continuous or discontinuous. Discontinuities in the adhesive 905 may be provided by apertures or holes (not shown) in the adhesive 905. The apertures or holes in the adhesive 905 may be formed after application of the adhesive 905 or by coating the adhesive 905 in patterns on a carrier layer, such as, for example, a side of the cover 125. Apertures or holes in the adhesive 905 may also be sized to enhance the MVTR of the adhesive 905 in some example embodiments.

The adhesive 905 can be disposed on a bottom side of the cover 125, and the adhesive 905 may pressed onto the cover 125 and epidermis 810 (or other attachment surface) to fix the dressing 110 in position and to seal the tissue interface 120 over the patient. In some embodiments, the adhesive 905 can be disposed only around edges of the cover 125.

FIGURE 9 also illustrates one example of a fluid conductor 910 and a dressing interface 915. As shown in the example of FIGURE 9, the fluid conductor 910 may be a flexible tube, which can be fluidly coupled on one end to the dressing interface 915. The dressing interface 915 may be an elbow connector. In some examples, the tissue interface 120 can be applied to the tissue site before the cover 125 is applied over the tissue interface 120. The cover 125 may include an aperture 920, or the aperture 920 may be cut into the cover 125 before or after positioning the cover 125 over the tissue interface 120. The position of the aperture 920 may be off-center or adjacent to an end or edge of the cover 125. In other examples, the aperture 920 may be centrally disposed. The dressing interface 915 can be placed over the aperture 920 to provide a fluid path between the fluid conductor 910 and the tissue interface 120. In other examples, the fluid conductor 910 may be inserted directly through the cover 125 into the tissue interface 120.

If not already configured, the dressing interface 915 may be disposed over the aperture 920 and attached to the cover 125. The fluid conductor 910 may be fluidly coupled to the dressing interface 915 and to the negative-pressure source 105.

Negative pressure from the negative-pressure source 105 can be distributed through the fluid conductor 910 and the dressing interface 915 to the tissue interface 120. The dressing 110 may be a bolster to aid in closing the tissue site 805. The tissue interface 120 may contract in response to the application of negative pressure. In some embodiments, the manifold layer 205 of the tissue interface 120 is configured to contract. For example, under an applied negative pressure, the manifold layer 205 may contract radially in all directions.

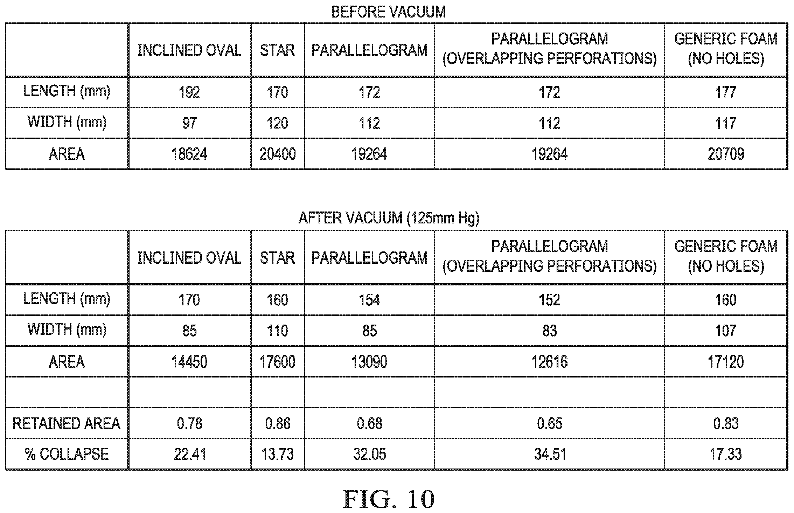

FIGURE 10 is a table illustrating an improved percentage of collapse of the tissue interface 120 according to this disclosure. Testing was performed on manifolds comprising a plurality of holes shaped like an oval, a star, a parallelogram, and a parallelogram with overlapping perforations. The testing measured the percent collapse of each of the manifolds under negative pressure (about -125 mm Hg). The percentage of collapse is generally between about 20% and 30% of the initial area of the manifold. However, the percentage of collapse of a manifold with holes shaped like a parallelogram and a manifold with holes shaped like a parallelogram with overlapping perforations in accordance with this disclosure resulted in a collapse of 32.05% and 35.51%, respectively, higher than any other tested shapes or patterns.

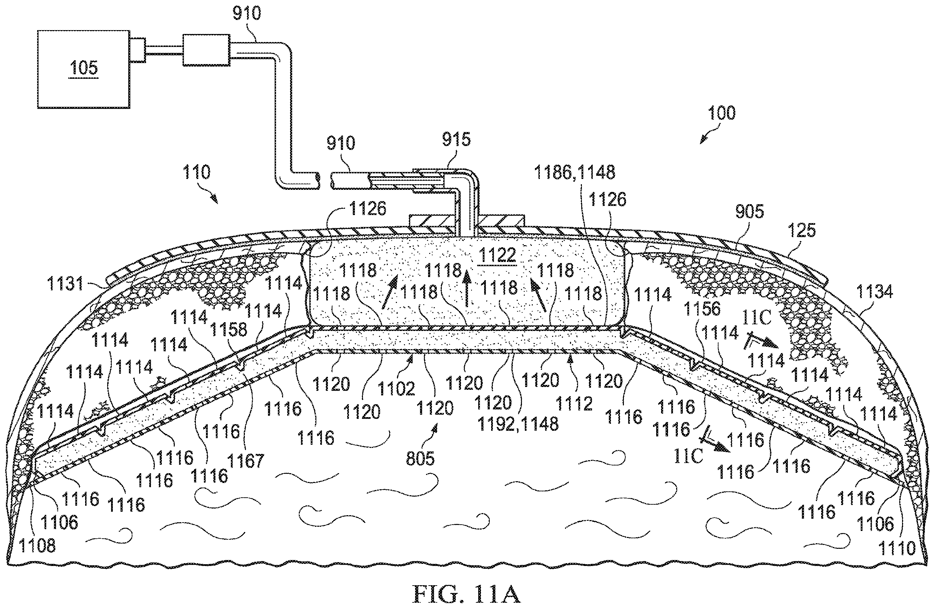

FIGURES 11A-11D relate to another exemplary embodiment of a therapy system 100, configured for use on an open-cavity, such as an abdominal cavity. In some embodiments, the therapy system 100 of FIGURES 11A-11D may include a dressing 110 having a treatment device 1102. For example, the tissue interface of the dressing 110 may include the treatment device 1102, in some embodiments. The open-cavity reduced-pressure therapy system 100 and the treatment device 1102 are for treating the tissue site 805 of a patient. The tissue site 805 may be the bodily tissue of any human, animal, or other organism, including bone tissue, adipose tissue, muscle tissue, dermal tissue, tissue, connective tissue, cartilage, tendons, ligaments, or any other tissue. In this illustrative embodiment, the tissue site 805 includes tissue in a body cavity, and in particular the abdominal cavity, and includes the abdominal contents or tissue that is proximate the abdominal cavity. Treatment of the tissue site 805 may include removal of fluids, e.g., exudate or ascites, protection of the abdominal cavity, or reduced-pressure therapy.

As shown, the treatment device 1102 is disposed within the abdominal cavity of the patient to treat the tissue site 805. The treatment device 1102 includes a plurality of encapsulated leg members 1106 that are supported by the abdominal contents, which make up a surface on which the plurality of leg members 1106 are placed. One or more of the plurality of encapsulated leg members 1106 may be placed in or proximate to a first paracolic gutter 1108, and one or more of the plurality of encapsulated leg members 1106 may be placed in or proximate to a second paracolic gutter 1110. The plurality of encapsulated leg members 1106 is coupled to a central connection member 1112, and there is fluid communication between the plurality of encapsulated leg members 1106 and the central connection member 1112. The plurality of encapsulated leg members 1106 and/or the central connection member 1112 may be formed with fenestrations 1114, 1116, 1118, 1120 (or other such passages or perforations) that allow fluids in the abdominal cavity to pass through. The fenestrations 1114, 1116, 1118, 1120 may take any shape, *e.g.,* circular apertures, rectangular openings, polygons, etc., but are presented in this illustrative embodiment as slits, or linear cuts. One or more fenestrations 1114, 1116, 1118, 1120 might be omitted in alternative embodiments.

A manifold pad 1122, which may be similar to the manifold described with respect to FIGURE 1, may distribute reduced pressure to the treatment device 1102. For example, the tissue interface 120 may comprise the manifold pad 1122 in conjunction with the treatment device 1102. A sealing member, such as cover 125, may provide a pneumatic seal over body-cavity opening 1126. One or more skin closure devices may be placed on a patient's epidermis 1134. Reduced pressure is delivered to the manifold pad 1122 through a dressing interface 915, which is coupled to a fluid conductor 910. A negative-pressure source 105 delivers reduced pressure to the fluid conductor 910.

The reduced pressure may be applied to the tissue site 805 to help promote removal of ascites, exudates, or other fluids from the tissue site 805. In some instances, reduced pressure may be applied to stimulate the growth of additional tissue. In some instances, only fluid removal may be desired. In the case of a wound at the tissue site 805, the growth of granulation tissue, removal of exudates, or removal of bacteria may help to promote healing of the wound. In the situation of a non-wounded or non-defective tissue, reduced pressure may be used in some instances to promote the growth of tissue that may be harvested and transplanted to another tissue site.

Reduced pressure may initially generate fluid flow in the manifold pad 1122, the fluid conductor 910, and proximate the tissue site 805. As the hydrostatic pressure around the tissue site 805 approaches the desired reduced pressure, the flow may subside, and the reduced pressure may be maintained.

The manifold pad 1122 in FIGURE 11A is proximate the central connection member 1112. The manifold pad 1122 may take many forms. Similar to the description above, the term "manifold" as used herein generally refers to a substance or structure that is provided to assist in applying reduced pressure to, delivering fluids to, or removing fluids from the tissue site 805. The manifold pad 1122 typically includes a plurality of flow channels or pathways that distribute the fluids provided to and removed from the tissue site 805 around the manifold pad 1122 and through the central connection member 1112. In one illustrative embodiment, the flow channels or pathways are interconnected to improve distribution of fluids provided or removed from the tissue site 805. The manifold pad 1122 may be a biocompatible material that is capable of being placed in contact with the tissue site 805 and distributing reduced pressure to the tissue site 805.

Examples of suitable materials for the manifold pad 1122 may include, without limitation, devices that have structural elements arranged to form flow channels, cellular foam, such as open-cell foam, porous tissue collections, liquids, gels and foams that include or cure to include flow channels. The manifold pad 1122 may be porous and may be made from foam, gauze, felted mat, silicone, polyvinyl alcohol, or any other material suited to a particular biological application. In one embodiment, the manifold pad 1122 is a porous foam and includes a plurality of interconnected cells or pores that act as pathways or flow channels. The porous foam may be a polyurethane or polyether, open-cell, reticulated foam, such as GRANUFOAM^{™} material available from Kinetic Concepts, Incorporated in San Antonio, Texas. Other embodiments might include "closed cells." These closed-cell portions of the manifold may contain a plurality of cells, the majority of which are not fluidly connected to adjacent cells. The closed cells may be selectively disposed in the manifold pad 1122 to prevent transmission of fluids through perimeter surfaces of the manifold pad 1122. In some situations, the manifold pad 1122 may also be used to distribute fluids such as medications, antibacterials, growth factors, and various solutions to the tissue site 805. Other layers may be included in or on the manifold pad 1122, such as absorptive materials, wicking materials, hydrophobic materials, and hydrophilic materials.

A material with a higher or lower density than GRANUFOAM^{™} material may be desirable depending on the application. Among the many possible materials, the following may be used: GRANUFOAM^{™} material, FOAMEX^{™} technical foam, a molded bed of nails structures, a patterned grid material such as those manufactured by Sercol Industrial Fabrics, 3D textiles such as those manufactured by Baltex of Derby, United Kingdom, a gauze, a flexible channel-containing member, a graft, or similar materials. In some instances, ionic silver may be added to the manifold pad 1122 by, for example, a micro bonding process. Other substances, such as anti-microbial agents, may be added to the manifold pad 1122 as well.

The cover 125 in FIGURE 11A is configured to be placed over the body-cavity opening 1126 and may be formed from any material capable of providing a pneumatic seal or fluid seal adequate for the open-cavity, reduced-pressure system 100 to hold a reduced pressure at the tissue site 805. The cover 125 may be used to secure the manifold pad 1122 on the central connection member 1112. The cover 125 may be impermeable or semi-permeable. The cover 125 is capable of maintaining reduced pressure at the tissue site 805 after installation of the cover 125 over the body-cavity opening 1126. The cover 125 may be a flexible over-drape or film formed from a silicone-based compound, acrylic, hydrogel or hydrogel-forming material, or any other biocompatible material that includes the impermeability or permeability characteristics as desired for applying reduced pressure to the tissue site 805.

The cover 125 may further include an attachment means 1131 to secure the cover 125 to the patient's epidermis 1134. The attachment means 1131 may take many forms; for example, an adhesive layer 1136 (e.g. with adhesive similar to that of FIGURE 9) may be positioned along a perimeter of the cover 125 or any portion of the cover 125 to provide, directly or indirectly, the pneumatic seal with the patient's epidermis 1134. The adhesive layer 1136 might also be pre-applied to the cover 125 and covered with a releasable backing, or member (not shown), that is removed at the time of application. In some embodiments, the adhesive layer 1136 may comprise the adhesive 905, or similar adhesive material.

The dressing interface 915 may be, as one example, a port or connector, which permits the passage of fluid from the manifold pad 1122 to the fluid conductor 910 and vice versa. For example, fluid collected from the tissue site 805 using the manifold pad 1122 and the treatment device 1102 may enter the fluid conductor 910 via the connector. In another embodiment, the open-cavity, reduced-pressure system 100 may omit the connector and the fluid conductor 910 may be inserted directly into the cover 125 and into the manifold pad 1122. The fluid conductor 910 may be a medical conduit or tubing or any other means for transportating a reduced pressure and fluid. The fluid conductor 910 may be a multi-lumen member for readily delivering reduced pressure and removing fluids. In one embodiment, the fluid conductor 910 is a two-lumen conduit with one lumen for reduced pressure and liquid transport and one lumen for communicating pressure to a pressure sensor.

Reduced pressure is generated and supplied to the fluid conductor 910 by the negative-pressure source 105. A wide range of reduced pressures may be generated or supplied by the negative-pressure source 105. In one embodiment, the range may include -50 to -300 mm Hg, and in another embodiment, the range may include -100 mm Hg to -200 mm Hg. In one illustrative embodiment, the negative-pressure source 105 includes preset selectors for -100 mm Hg, -125 mm Hg, and -150 mm Hg. The negative-pressure source 105 may also include a number of alarms, such as a blockage alarm, a leakage alarm, or a battery-low alarm.

A number of different devices may be added to a medial portion of the fluid conductor 910. For example, the device might be a fluid reservoir, or canister collection member, a pressure-feedback device, a volume detection system, a blood detection system, an infection detection system, a filter, a port with a filter, a flow monitoring system, a temperature monitoring system, etc. Multiple devices might be included. Some of these devices, *e.g.,* the fluid collection member, may be formed integral to the negative-pressure source 105. For example, a reduced-pressure port on the negative-pressure source 105 may include a filter member (not shown) that includes one or more filters and may include a hydrophobic filter that prevents liquid from entering an interior space of the negative-pressure source 105.

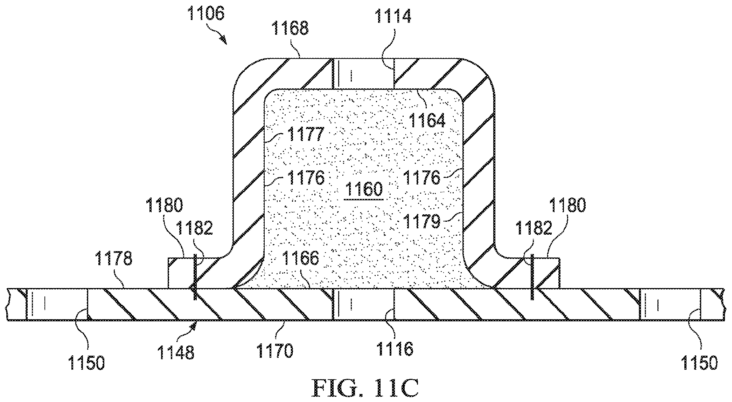

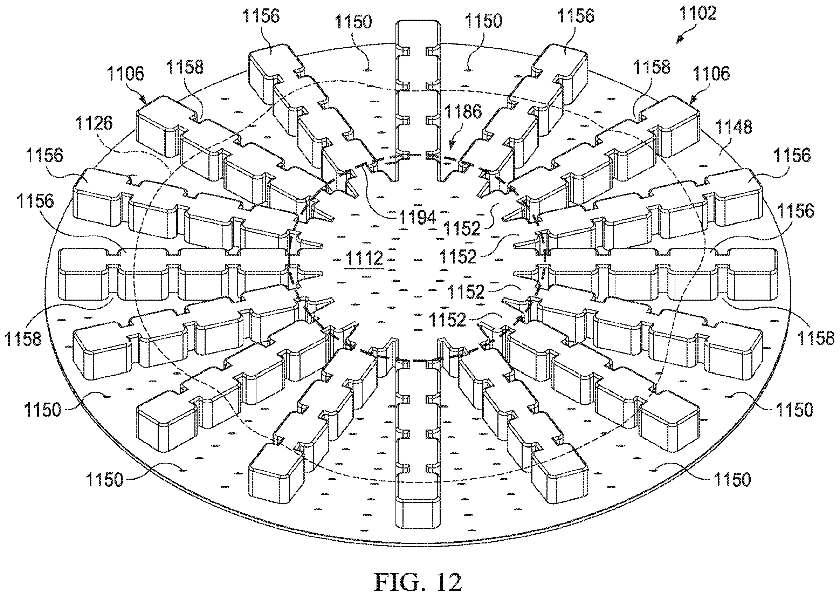

Referring now to FIGURES 11A, 11C, and 12, the treatment device 1102 can include a non-adherent drape 1148. The non-adherent drape 1148 may be formed of a non-adherent material that inhibits tissue adhesion to the non-adherent drape 1148. In some embodiments, the non-adherent drape 1148 may be similar to the contact layer or the film layer 220 of FIGURE 2B. In some embodiments, the non-adherent drape 1148 may comprise a substantially fluid impermeable layer, which may be the film layer 220. In some embodiments, the non-adherent drape 1148 is formed from a breathable polyurethane film. The non-adherent drape 1148 can include a plurality of openings, apertures, passages, perforations, or fenestrations 1150. The fenestrations 1150 can take a variety of shapes, such as circular openings, rectangular openings, polygon-shaped openings, etc., but are shown in FIGURE 2 as slits, or linear cuts. Depending on the particular application of treatment device 1102, the desired fluid flow and/or pressure delivery, or other system parameters, the fenestrations can have different sizes.

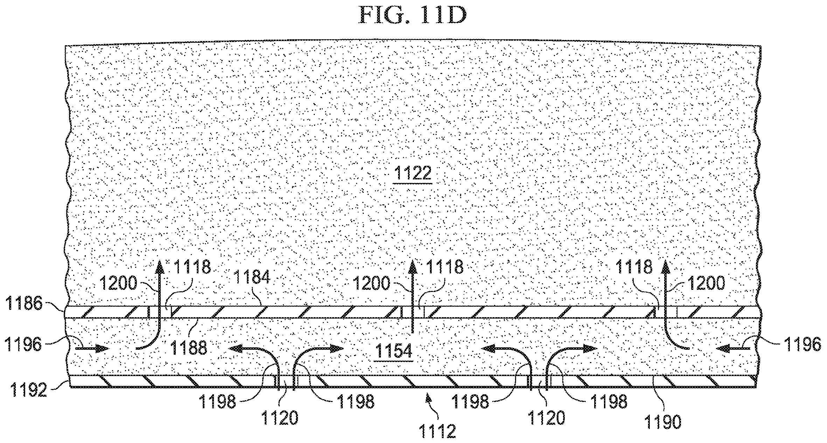

Referring to FIGURES 11A, 11D, and 12, the treatment device 1102 includes the central connection member 1112 to which the plurality of encapsulated leg members 1106 are coupled. The central connection member 1112 includes a connection manifold member 1154 that is encapsulated by a first connection encapsulation member 1186, which may be referred to as a first non-adherent drape 1186, and a second connection encapsulation member 1192, which may be referred to as a second non-adherent drape 1192. A portion of the central connection member 1112 can fluidly couple at leg coupling areas 1152 to permit fluid communication between the central connection member 1112 and the plurality of encapsulated leg members 1106. The first and the second connection encapsulation member or non-adherent drape 1186, 1192 may be defined by a single piece of material or, as illustrated, more than one sheet of material. For example, the non-adherent drape 1148 may be used as a single sheet or may include the first non-adherent drape 1186 and the second non-adherent drape 1192.

The central connection member 1112, as discussed above, can fluidly communicate with manifold pad 1122. In one aspect, fenestrations 1118, similar to the fenestrations discussed above, can permit fluid communication. Additionally, or alternatively, a portion or portions of the first connection encapsulation member or non-adherent drape 1186 can be exposed to manifold pad 1122.

Referring again to FIGURES 11A-11D, each of the plurality of encapsulated leg members 1106 can include a leg manifold member 1160, which may be a single manifold member that runs between leg modules 1156 and/or central connection member 1112, or individual manifold components. The leg manifold member 1160 is disposed within an interior portion 1162 of each of the encapsulated leg members 1106. Each leg manifold member 1160 has a first side 1164 and a second, inward-facing (patient-facing) side 1166.

In some embodiments, the connection manifold member 1154 and one or more of the leg manifold members 1160 extending from the connection manifold member 1154 may form a treatment manifold 1167. The treatment manifold 1167 including the leg manifold members 1160 and the connection manifold member 1154 may comprise or be formed of a foam or a manifold material similar or analogous to the manifold materials described herein for the manifold pad 1122 or the manifold layer 205.

In some embodiments, the non-adherent drape 1148 may surround the treatment manifold 1167. For example, the non-adherent drape 1148 may be coupled around the leg manifold members 1160 and the connection manifold member 1154 to provide the plurality of encapsulated leg members 1106 in fluid communication with the central connection member 1112. In some embodiments, the non-adherent drape 1148 may include the first non-adherent drape 1186 and the second non-adherent drape 1192 and the treatment manifold 1167 may be positioned as a layer between the first non-adherent drape 1186 and the second non-adherent drape 1192. The first non-adherent drape 1186 may be coupled to the second non-adherent drape 1192 to provide the plurality of encapsulated leg members 1106 in fluid communication with the central connection member 1112.

In one embodiment, one or more of the plurality of leg manifold members 1160 can have different material properties or structures. For example, different flow rates may be desired in different encapsulated leg members 1106. In one aspect, different manifold materials or manifold properties, different manifold sizes, manifold compression, the use flow restricting material structures, and/or or valves can provide different flow rates of fluid through the encapsulated leg members and/or central connection member.

In one aspect, a first leg encapsulating member 1168, which can be formed with fenestrations 1114, is disposed on the first side 1164 of the leg manifold member 1160. A second leg encapsulating member 1170, which can include fenestrations 1116, is disposed on the second, inward-facing side 1166 of the leg manifold member 1160. The first leg encapsulating member 1168 and the second leg encapsulating member 1170 may be a portion of the non-adherent drape 1148. In some embodiments, the first leg encapsulating member 1168 may be a portion of the first non-adherent drape 1186 and the second leg encapsulating member 1170 may be a portion of the second non-adherent drape 1192. In some embodiments, the encapsulated leg members 1106 can be mated with one another, for example, via the drape 1148. In some embodiments, the encapsulated leg members 1106 can be independently movable with respect to one another with the exception of their proximal end adjacent to the central connection member 1112. For example, the encapsulated leg members 1106 need not be connected to one another. In another embodiment, a portion of the material connecting the encapsulated leg members 1106, *e.g.,* the non-adherent drape 1148 between adjacent encapsulated leg members 1106, is expandable (*e.g.,* a stretchable, flexible, deformable, and/or elastic material) and permits movement of individual encapsulated leg members 1106 relative to one another.

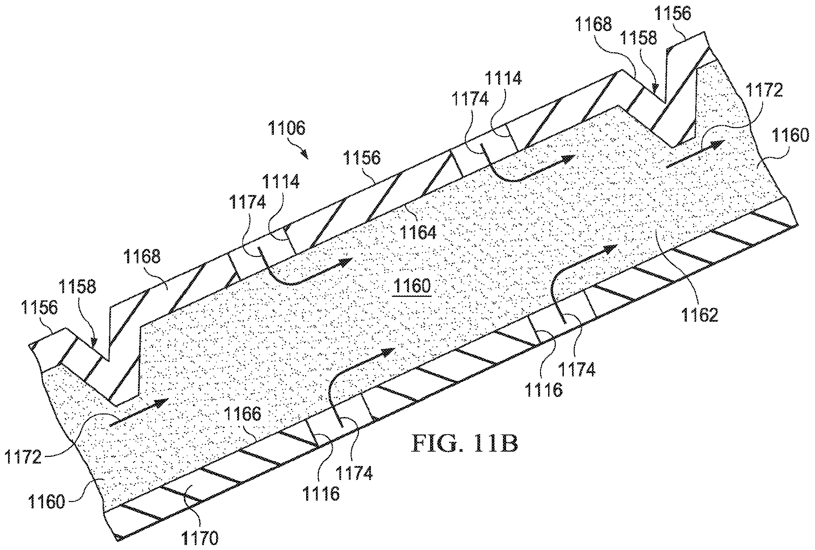

As shown in the longitudinal cross section of FIGURE 11B by arrows 1172, fluid can flow from leg modules 1156 towards the central connection member 1112. As shown by arrows 1174, the fluid is able to enter fenestrations 1114 and 116 and flow into the leg manifold member 1160 and then flow toward the central connection member 1112 as represented by arrows 1172.

In plan view, the encapsulated leg members 1106 may take a number of different shapes, such as elongate shapes, rectangular, elliptical, etc. In one aspect, the encapsulated leg members 1106 may include leg modules 1156. Adjacent leg modules 1156 are fluidly coupled to each other and have a manipulation zone 1158 between them. In one aspect, the manipulation zone includes a weakened or perforated area to facilitate sizing of the device. For example, a clinician can cut through a leg module to size the device. By pulling on the partially cut leg module the manifold can be torn away at the next manipulation zone. In one aspect, the recessed shape of the manipulation zone 1158 can inhibit accidental removal of additional leg modules. Additionally, or alternatively, the outer portion of the leg modules can be fixed to the device to inhibit unwanted manifold removal.

The encapsulated leg members 1106 may also have various dimensions. If the longer dimension, *e.g.,* lengthwise or longitudinal dimension, of the encapsulated leg 106 is _{L1} and the width is wi, then the aspect ratio is given by _{L1}/_{W1}. The aspect ratio may be 8.0, 7.0, 6.0, 5.0, 4.0, 3.0, 2.0 or any number in between. Moreover, other aspect ratios are possible. Generally, the width _{W1} of the encapsulated leg member will be greater than a width _{W2} of the central connection member 1112, *i.e.,* _{W2} > _{W1}. For example, in one illustrative embodiment, the encapsulated leg members 1106 are approximately 270 mm long, 60 mm wide (wi), and 10 mm thick, and the central connection member has a width parallel to the first width (_{W1}) of about 130 mm (_{W2}). Thus, in that illustrative example, the aspect ratio of the encapsulated leg 106 is approximately (270/60) or 4.5. In this same illustrative embodiment, the manipulation zones 158 have a width of about 10 mm.

Referring to FIGURE 11C, a lateral cross section of a portion of the encapsulated leg member 1106 is presented. As before, it can be seen that the first side 1164 of the leg manifold member 1160 is covered with the first leg encapsulating member 1168, and that the second, inward-facing side 1166 of the leg manifold member 1160 is covered by the second leg encapsulating member 1170, which in this instance is a portion of the non-adherent drape 1148. Thus, in this illustrative embodiment, the fenestrations 1116 may be some of the plurality of fenestrations 1150 in the non-adherent drape 1148. In this illustrative embodiment, peripheral edges 1176 of the leg manifold member 1160 are also covered by a portion of the first leg encapsulating member 1168. The peripheral edges 1176 include a first lateral edge 1177 and a second lateral edge 1179. The first leg encapsulating member 1168 covers the first side 1164 and the peripheral edges 1176 and extends onto a first surface 1178 of the non-adherent drape 1148 and forms extensions 1180. The extensions 1180 have been coupled to the second leg encapsulating member 1170 by welds 1182. The first leg encapsulating member 1168 may, however, be coupled to the second leg encapsulating member 1170 using any known technique, including welding (*e.g.,* ultrasonic or RF welding), bonding, adhesives, cements, etc.

In some embodiments, at least a portion of a longitudinal length of at least one of the encapsulated leg members 1106 is configured to contact the tissue site 805. Further, in some embodiments, the fenestrations 1116 may be positioned along the longitudinal length of at least one of the encapsulated leg members 1160.

Referring again to FIGURE 11D and FIGURE 12, the central connection member 1112 includes the connection manifold member 1154 that is encapsulated within the first connection encapsulation member 1186, which has fenestrations 1118. The first connection encapsulation member 1186 is disposed on a first side 1188 of the connection manifold member 1154. The second connection encapsulation member 1192 is disposed on a second, inward-facing side 1190 of the connection manifold member 1154. The second connection encapsulation member 1192 is formed with fenestrations 1120. The first connection encapsulation member 1186 has a peripheral zone or edge 1194 as shown in FIGURE 2. In a similar fashion, the second connection encapsulation member 1192 has a peripheral zone or edge (not explicitly shown) that lines up with the peripheral edge 1194. The peripheral edge 1194 of the first connection encapsulation member 1186 is coupled to peripheral edge of the second connection encapsulation member 1192, except at the leg coupling areas 1152 in order to allow fluid within the plurality of encapsulated leg members 1106 to flow into the connection manifold member 1154 as suggested by arrows 1196 in FIGURE 11D. Fluid may also enter directly into the connection manifold member 1154 by flowing through fenestrations 1120 as suggested by arrows 1198. The manifold pad 1122 is disposed proximate to the first connection encapsulation member 1186, and when a reduced pressure is applied to the manifold pad 1122, the reduced pressure causes fluid to flow from the connection manifold member 1154 through fenestrations 1118 and into the manifold pad 1122 as suggested by arrows 1200. The fluid continues to flow in the direction of the dressing interface 915 through which the fluid is removed to the fluid conductor 910.

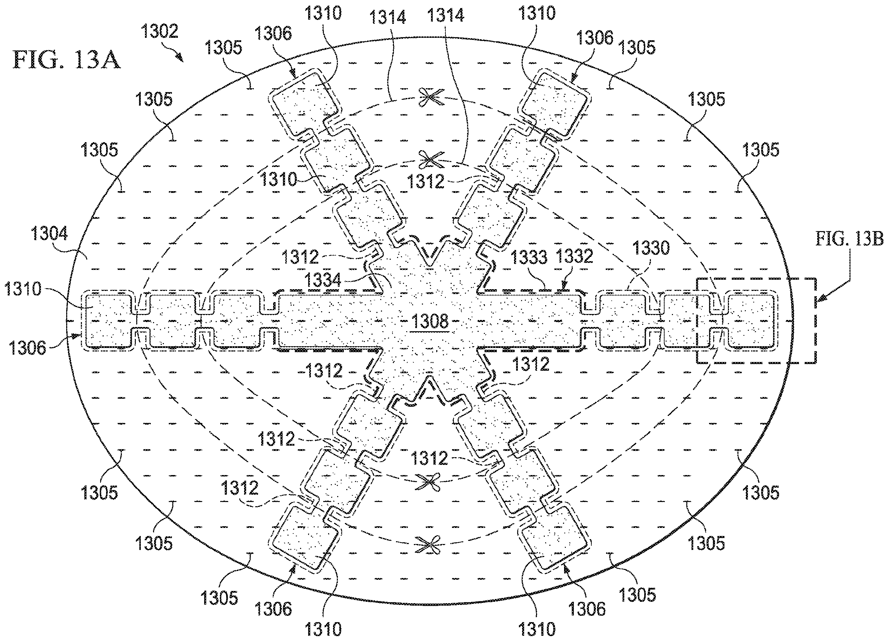

Referring to FIGURES 11A-11D and 12, in operation, the illustrative open-cavity, reduced-pressure system 100 may be used by first sizing the treatment device 1102 as will be explained further below in connection with FIGURE 13A. The non-adherent drape 1148 with the plurality of encapsulated leg members 1106 is disposed within the abdominal cavity through the body-cavity opening 1126 and is distributed against the abdominal contents; this may include placing at least one encapsulated leg member 1106 in or proximate the first paracolic gutter 1108, the second paracolic gutter 1110, or behind the liver, etc. Once the treatment device 1102 has been distributed, the manifold pad 1122 is placed adjacent a first side 1184 of the first connection encapsulation member 1186. The cover 125 may then be applied over the body-cavity opening 1126 to provide a pneumatic seal over the body-cavity opening 1126.

In addition to the cover 125, the body-cavity opening 1126 may be further closed or reinforced using mechanical closing means, *e.g.,* staples, or using a reduced-pressure closure system. The cover 125 may be applied in a number of ways, but according to one illustrative embodiment, the releasable backing member that is on the adhesive layer 1136 of the cover 125 is removed and then the cover 125 is placed against the patient's epidermis 1134 about the body-cavity opening 1126. The dressing interface 915 is then coupled or attached to the cover 125 such that reduced pressure can be delivered by the dressing interface 915, through the cover 125, and to the manifold pad 1122 and the central connection manifold member 1154. The fluid conductor 910 is fluidly coupled to the dressing interface 915 and to the reduced-pressure port on the negative-pressure source 105.

The negative-pressure source 105 is activated and thereby provides reduced pressure into the fluid conductor 910, which delivers the reduced pressure to the dressing interface 915 and into the manifold pad 1122 and the central connection manifold member 1154. The manifold pad 1122 distributes the reduced pressure and draws fluid through fenestrations 1118 from the connection manifold member 1154. The connection manifold member 1154 draws fluid from the abdominal cavity through fenestrations 1120 and pulls fluid from the plurality of encapsulated leg members 1106 as suggested by arrows 196. Fluid from the abdominal cavity flows into the plurality of encapsulated leg members 1106 through fenestrations 1114 on the first leg encapsulating member 1168 and through fenestrations 1116 on the second leg encapsulating member 1170 and then flows through the leg as suggested by arrows 1172 towards the connection manifold member 1154. The fluid then flows through the manifold pad 1122, the dressing interface 915, and into the fluid conductor 910.

Referring now to FIGURES 13A-13C, another illustrative embodiment of an open-cavity, reduced-pressure treatment device 1302 is presented. The open-cavity, reduced-pressure treatment device 1302 is analogous in most respects to the treatment device 1102 of FIGURES 11A-11D. The open-cavity, reduced-pressure treatment device 1302 has a non-adherent drape 1304 (which may be similar to that 1148 in FIGURE 11A), a plurality of encapsulated leg members 1306, and a central connection member 1308. In this particular illustrative embodiment, the non-adherent drape 1304 is formed generally with an oval or arcuate shape. The non-adherent drape 1304 is formed with a plurality of fenestrations 1305, which may be similar to the fenestrations 1114, 1116, 1118, 1120, and 1150, for example. The non-adherent drape 1304 forms the second leg encapsulating member (see by analogy second leg encapsulating member 1170 in FIGURE 11B) and the second connection encapsulation member (see by analogy 1192 in FIGURE 11D). As such, the plurality of fenestrations 1305 serves as flow channels for the plurality of encapsulated leg members 1306 and the central connection member 1308 on the second, inward-facing side. The non-adherent drape 1304 could also be used on the first side of the plurality of encapsulated leg members 1306 and the central connection member 1308.

Each of the encapsulated leg members 1306 may be formed with a plurality of leg modules 1310 with manipulation zones 1312 between the plurality of leg modules 1310. As with the manipulation zone 1158 in FIGURES 11A-D, the manipulation zones 1312 facilitate movement of the plurality of encapsulated leg members 1306 within the body cavity and provide an easier location at which to cut the plurality of encapsulated leg members 1306 when the open-cavity, reduced-pressure treatment device 1302 is being sized for a particular application. In this regard, visual indicia 1314 may be added on the non-adherent drape 1304 to help the healthcare provider know where to cut the non-adherent drape 1304 for different sizes of application within the cavity. The visual indicia 1314 may comprise cut lines, lines of sizing perforations, or graduations that preferably run through the manipulation zones 1312. The manipulation zones 1312 provide a convenient and easy location for cutting the open-cavity, reduced-pressure treatment device 1302.

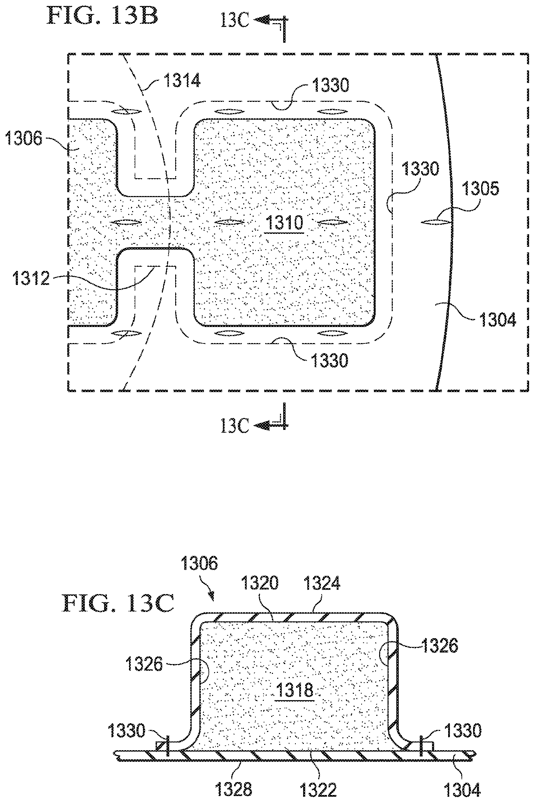

Referring to FIGURE 13C, a lateral cross section of a portion of an encapsulated leg member 1306 is presented. The plurality of encapsulated leg members 1306 are formed with a leg manifold member 1318 having a first side 1320 and a second, inward-facing (patient-facing) side 1322. A first leg encapsulating member 1324 covers the first side 1320 of the leg manifold member 1318 and covers a lateral zone or edge 1326 of the leg manifold member 1318. The second, inward-facing side 1322 of the leg manifold member 1318 is covered by a second leg encapsulating member 1328, which in this embodiment is a portion of the non-adherent drape 1304. The first leg encapsulating member 1324 is coupled to the second leg encapsulating member 1328 by any means known in the art, such as by welding (*e.g.,* ultrasonic or RF), bonding, adhesives, cements, etc. In this illustrative embodiment, the first leg encapsulating member 1324 and the second leg encapsulating member 1328 are coupled by a weld 1330. Referring to FIGURE 13B, the weld 1330 is shown along the perimeter of the plurality of leg modules 1310.

Referring again to FIGURE 13A, the central connection member 1308 is formed analogously to the central connection member 1112 in FIGURE 12. A first connection encapsulation member 1334 and a second connection encapsulation member of the central connection member 1308 are coupled along a peripheral edge 1332 using a weld 1333 or another coupling technique, such as those previously mentioned. The peripheral edge 1332 is not sealed, however, proximate each of the encapsulated leg members 1306 in order to provide a channel for fluid to flow from the plurality of encapsulated leg members 1306 into the central connection member 1308.

According to one illustrative approach to constructing the open-cavity, reduced-pressure treatment device 1302, the non-adherent drape 1304, which is already formed with the plurality of fenestrations 1305 and that has visual indicia 1314 is provided. The leg manifold member 1318 is disposed adjacent the non-adherent drape 1304. The central connection manifold 1308 is disposed adjacent to the leg manifold member 1318 or may be formed integral with leg manifold member 1318. The first connection encapsulation member 1334 is placed on the central connection member 1308, and the first leg encapsulating member 1324 is placed over the leg manifold member 1318. The first connection encapsulation member 1334 and the first leg encapsulating member 1324 may be formed from an integral sheet. Next, the welds 1330 and 1333 are applied.

In an alternative embodiment for manufacturing an open-cavity, reduced-pressure treatment device, a first non-adherent drape 1304, which includes fenestrations, may be provided and the leg manifold member 1318 and the central connection manifold 1308 disposed on the first non-adherent drape 1304. A second non-adherent drape, which has fenestrations, is placed over the first non-adherent drape 1304, the leg manifold member 1318, and the central connection manifold 1308. Next, a plurality of welds (*e.g.,* thermal or RF or another coupling techniques used) are made, such as with the welds 1330. The first non-adherent drape 1304 and the second non-adherent drape may be cut to size before or after assembly. By using two drapes, the first non-adherent drape 1304 and the second non-adherent drape may provide better distribution of reduced pressure and may ease the manufacturing process.

The fenestrations may be formed before or after assembly. The perimeter of the first non-adherent drape 1304 and the second non-adherent drape may be welded. Other points may be welded between the drapes to form a single unit. In another alternative embodiment, the drapes may initially be placed and welded without fenestrations, and then fenestrations added to the drapes so that the fenestrations align. The fenestrations might also be formed using an electrical member that cuts and seals at the same time to form aligned, "button hole" fenestrations through the two drapes.

Another illustrative embodiment for using an open-cavity, reduced-pressure treatment device or system according to this disclosure will now be presented. The system may be particularly suitable for temporary bridging of abdominal wall openings where primary closure may not be readily possible and or repeat abdominal entries are necessary. The illustrative system described here may be used with open abdominal wounds, with exposed viscera, including but not limited to abdominal compartment syndrome. Before applying the system, typically hemostasis should be achieved.

In deploying the open-cavity, reduced-pressure treatment system, the reduced-pressure treatment device preferably covers all exposed viscera and preferably separates completely the viscera from contact with the abdominal wall. For example, the lower surface of the reduced pressure treatment device, such as drape 1148, can be sized and shaped to permit coverage. The reduced-pressure treatment device may be placed over the omentum or exposed internal organs, and carefully tucked between the abdominal wall and internal organs. In doing so, the healthcare provider may use the reduced-pressure treatment device to completely separate the abdominal wall from the internal organs.

To prepare for deployment of the system, any sharp edges or bone fragments are eliminated from wound area or covered. The abdominal wound is irrigated and the periwound area cleaned. The periwound tissue at the epidermis is typically dried before further application.

The reduced-pressure treatment device is then sized by determining the appropriate size and cutting. The reduced-pressure treatment device is initially unfolded in a sterile field. Either side of the reduced-pressure treatment device may be placed on the omentum or viscera. The reduced-pressure treatment device is gently placed over the open abdominal cavity. The orientation of the reduced-pressure treatment device for the specific application is determined. If the reduced-pressure treatment device will be placed around tubes, drains or the falciform ligament, the reduced-pressure device is cut only between the plurality of encapsulated leg members. The reduced-pressure treatment device is placed in the proper orientation before cutting.

The reduced-pressure treatment device is then folded to size and used that way or may be cut. The healthcare provider holds the reduced-pressure treatment device by the edge and slightly lifts the reduced-pressure treatment device. The reduced-pressure treatment device is slowly lowered into the paracolic gutter with one hand and the other hand is used to gently and evenly work the reduced-pressure treatment device down. The healthcare provider folds any excess portions of the reduced-pressure treatment device up and over onto itself. The healthcare provider continues to place the reduced-pressure treatment device between abdominal wall and internal organs throughout the abdominal compartment. The healthcare provider preferably provides full coverage of all viscera. The reduced-pressure treatment device may then be cut as needed for sizing outside of the wound.

To size the device, the reduced-pressure treatment device can be cut through center of one of the large manifold squares, or leg modules, using sterile scissors. In this illustrative embodiment, the cut may not be made through the manipulation zone, but through the leg module. The healthcare provider may then pinch the remaining half of the foam square, or leg module, and the adjacent, inboard manipulation zone through the encapsulating member with one hand and pull the manifold material. The manifold material in the leg module and the manipulation zone will separate at the next square, or leg module. This will ensure that edges of the reduced-pressure treatment device cover the otherwise exposed manifold edge. The manifold material, *e.g.,* foam, preferably does not contact organs.

The manifold pad that is to be placed on top of the central connection member is next prepared. In this embodiment, the manifold may be a perforated foam manifold that has perforations to help tear the manifold to the desired size. The manifold pad preferably fits directly over the treatment device while still being in contact with wound edges. The manifold pad should not contact intact skin. Two or more manifolds may be used in some instances. Then, the sized manifold pad is gently placed into the wound cavity over the treatment device. The healthcare provider preferably takes care to avoid the manifold pad going below the level of the abdominal incision or wound.

A drape, cover, or over-drape, is then applied. To apply the drape, a backing may be removed from an adhesive layer on one side of the drape and the drape may be applied. The drape covers the manifold and a portion of intact epidermis. Preferably the drape covers at least an 8-10 centimeter border of intact periwound tissue. Additional drape material may be added to seal any difficult areas.

The dressing interface, or interface pad, is then added. The healthcare provider chooses an application site. The site is chosen to optimize fluid flow as well as to facilitate easy tubing positioning. The healthcare provider pinches the drape and cuts a 2.5 cm hole (preferably not a slit) through the drape. The dressing interface pad, which may have a central disc and a surrounding outer adhesive skirt, is applied. The dressing interface pad is applied by removing backing layers on an inward-facing surface of the interface pad to expose an adhesive. The dressing interface pad opening in the central disc is placed directly over the hole in the drape. Pressure is gently applied on the central disc and the outer skirt to ensure complete adhesion of the dressing interface pad. One or more stabilization layers may then be removed from a first side of the skirt. The system is now ready for the application of reduced pressure.

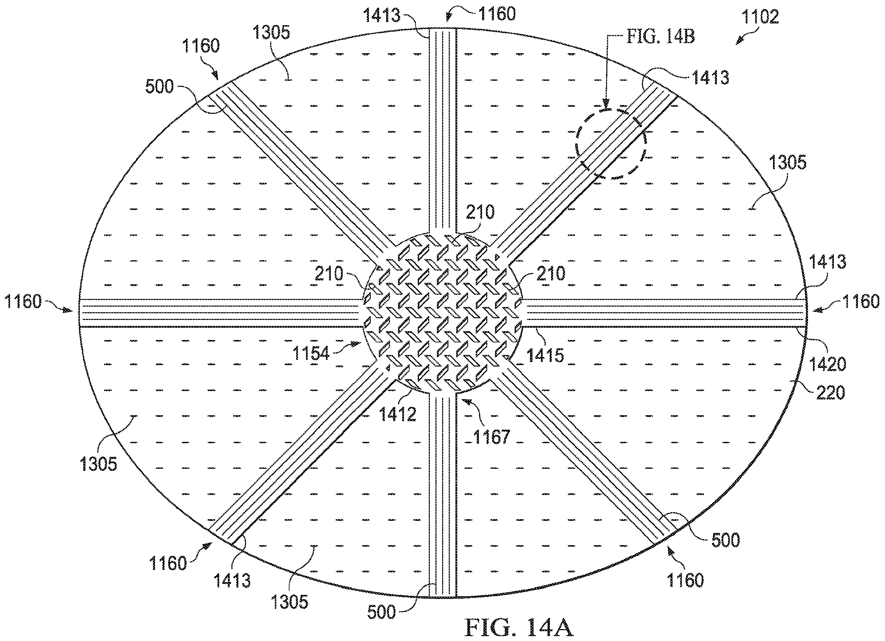

FIGURES 14A-F show yet another embodiment of the treatment device 1102, illustrating additional details that may be associated with some embodiments. The exemplary embodiment shown in FIGURES 14A-F may be similar in many respects to the embodiments shown in FIGURES 11A-13C, except that the treatment manifold 1167 (e.g. the central connection manifold member 1154 and/or the leg manifold members 1160) may not be encapsulated. Rather than having non-adherent drape (e.g. film) entirely around and/or encasing the treatment manifold 1167, the treatment device 1102 of FIGURES 14A-F may only have a single film layer 220 or non-adherent drape in contact with a first surface 1405 of the treatment manifold 1167, and a second surface 1410 of the treatment manifold 1167 (e.g. opposite the first surface 1405) may be exposed and/or uncovered by film or other contact layer or non-adherent drape. In some embodiments, to address tissue in-growth concerns which might arise from having an exposed manifold surface, the treatment manifold 1167 may be configured to minimize tissue in-growth. For example, the treatment manifold 1167 may comprise or consist essentially of a closed-cell foam, such as, for example, those manufactured by Zotefoams, Inc. of Walton, Kentucky, U.S.A., including the Azote, Plastazote, Evazote, Supazote, and Zotek grades.

For example, as shown in FIGURES 14A-F, the treatment device 1102 may comprise: a central fluid hub 1412, which may include or may be a connection manifold member 1154; a plurality of elongate members 1413, each of which may include or may be a leg manifold member 1160; and only one substantially fluid impermeable layer (such as film layer 220), which is attached to the first surface 1405 of each of the leg manifold members 1160. In some embodiments, the fluid impermeable layer may also be attached to the first surface of the connection manifold member 1154. In some embodiments, the plurality of elongate members 1413 (e.g. the leg manifold members 1160) may extend outward from the central fluid hub 1412 (e.g. the centrally located connection manifold member 1154), and each of the plurality of leg manifold members 1160 may be in fluid communication with the connection manifold member 1154. In some embodiments, the substantially fluid impermeable layer (e.g. the film layer 220) may comprise a plurality of fenestrations 1305 (e.g. configured to allow fluid communication through the liquid impermeable film layer 220 between the manifold and the tissue site).

In some embodiments, the treatment manifold 1167 (e.g. the connection manifold member 1154 and/or the leg manifold members 1160) may be configured to distribute negative pressure (e.g. along one or more external surfaces) while substantially minimizing tissue in-growth during negative-pressure therapy. The configuration of the treatment manifold 1167 may allow for effective distribution of negative pressure and/or drawing of fluid from the tissue site under negative pressure, even in instances when the treatment manifold 1167 is formed of a material, such as closed-cell foam, which may not be inherently effective for manifolding, negative pressure distribution, and/or drawing of fluid. For example, the connection manifold member 1154 and the leg manifold members 1160 each may comprise or consist essentially of closed-cell foam (such as, for example, those manufactured by Zotefoams, Inc. of Walton, Kentucky, U.S.A., including the Azote, Plastazote, Evazote, Supazote, and Zotek grades), which may be configured with fluid pathways to allow for negative pressure distribution. In some embodiments, the treatment manifold 1167 may be formed of some different manifold material other than closed-cell foam, with the different manifold material being configured to minimize tissue in-growth during negative-pressure therapy. For example, the different manifold material may be felted foam. Some alternate embodiments of the treatment device 1102 may comprise a second film layer (not shown, but which may be similar to the first film layer 220) attached to the second surface 1410 of the treatment manifold 1167. Some other alternate embodiments of the treatment device 1102 (not shown) may not include any film layer, but may comprise or consist essentially of only the treatment manifold 1167 configured to minimize tissue in-growth. For example, no film may be attached to the treatment manifold 1167 in such embodiments.

As shown in FIGURES 14A-F, the treatment manifold 1167 may not be encapsulated. For example, the treatment manifold 1167 may only be covered by film on one surface (e.g. the single film layer 220 may only attach to the first surface 1405), and the second surface 1410 of the treatment manifold 1167 may be uncovered (e.g. may not contact a film layer and/or may be configured to directly contact tissue). The second surface 1410 of the treatment manifold 1167 may be opposite the first surface 1405, for example with the first surface 1405 and the second surface 1410 separated by a thickness of the treatment manifold 1167 (e.g. disposed approximately parallel and separated by the thickness of the treatment manifold 1167). In some embodiments, the leg manifold members 1160 and the central connection manifold member 1154 may all be located within the same plane. For example, each of the leg manifold members 1160 may extend outward from the central connection manifold member 1154 in substantially the same plane as the central connection manifold member 1154. In some embodiments, the thickness of the treatment manifold 1167 may be about 4-6 mm.

In some embodiments, each leg manifold member 1160 may comprise a proximal end 1415 and a distal end 1420, and the proximal end 1415 of each leg manifold member 1160 may be attached to and in fluid communication with the central connection manifold member 1154 such that, for example, fluid communication therebetween is through the proximal end 1415 of the leg manifold member 1160. In some embodiments, the leg manifold members 1160 may not contact each other (e.g. there may be no contact between adjacent leg manifold members 1160). Rather, there may be space between adjacent leg manifold members 1160. In some embodiments, the plurality of leg manifold members 1160 may extend outward from central connection manifold member 1154 to be spaced apart, with space between adjacent leg manifold members 1160 increasing as the leg manifold members 1160 extend outward. In some embodiment, the plurality of leg manifold members 1160 may comprise 6 or 8 leg manifold members 1160. In some embodiments, the leg manifold members 1160 may be approximately equal spaced around the central connection manifold member 1154. For example, adjacent leg manifold members 1160 may be spaced apart to extend at an angle of approximately 45 degrees. In some embodiments, each of the leg manifold members 1160 may be shaped to extend substantially straight away from the central connection manifold member 1154.

In some embodiments, the treatment manifold 1167 may be configured to distribute negative-pressure (e.g. from the central connection manifold member 1154 outward through the leg manifold members 1160), and thereby to draw fluid from the tissue site, through the leg manifold members 1160, to the central connection manifold member 1154, and out of the central connection manifold member 1154 towards the negative-pressure source. And as noted above, the treatment manifold 1167 may also be configured to minimize in-growth of tissue during extended wear or usage (e.g. for at least 7 days, at least 10 days, or at least 14 days), for example by being formed of closed-cell foam. For example, the treatment manifold 1167 may comprise a plurality of channels 500, which may be surface or other channels similar to those shown in FIGURES 5A-7, and a plurality of through-passages, such as the through-holes 1425, as discussed in more detail below, which may jointly allow effective manifolding of negative pressure and fluid flow via the treatment manifold 1167. In some embodiments, the plurality of channels 500 may be disposed on at least one of the first surface 1405 and second surface 1410 of the treatment manifold 1167. In other embodiments, the channels 500 may be disposed on both the first surface 1405 and the second surface 1410 of the treatment manifold 1167. In some embodiments, at least some of the through-passages may intersect at least some of the channels 500. For example, in some embodiments each of the through-passages in the leg manifold members 1160 may intersect at least one of the plurality of channels 500. In some embodiment, each of the through-passages in the leg manifold members 1160 may intersect two channels 500, for example a channel 500 on the first surface 1405 and a channel 500 on the second surface 1410 of the treatment manifold 1167, with the through-passage extending through the thickness of the treatment manifold 1167 between the first surface 1405 and the second surface 1410.

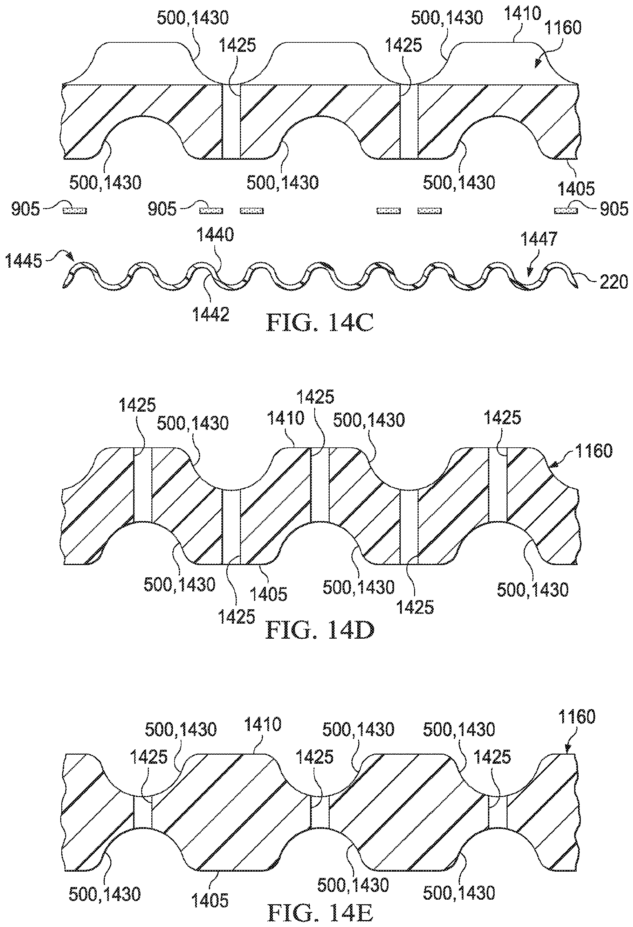

In some embodiments, the channels 500 on the first surface 1405 may be offset from the channels 500 on the second surface 1410, as shown in FIGURE 14D. For example, the channels 500 on the first surface 1405 may not align (e.g. may be out of alignment across the thickness of the treatment manifold 1167) with the channels 500 on the second surface 1410. In some embodiments, the channels 500 on the first surface 1405 may not be located directly across from the channels 500 in the second surface 1410, through the thickness of the treatment manifold 1167. In some embodiments, the channels 500 on the first surface 1405 (e.g. the center or deepest portion of the channels 500) may not overlap with the channels 500 on the second surface 1410 (e.g. the center or deepest portion of the channels 500), but may be off-center or disposed at an angle through the thickness of the treatment manifold 1167. In some embodiments, the maximum depth of the channels 500 on the first surface 1405 may correspond to the minimum depth of the channels 500 on the second surface 1410 (e.g. the portion of the second surface 1410 with no channel depth), and vice versa. In some embodiments, this offset may allow the thickness of the treatment manifold 1167 to be approximately consistent across the treatment manifold 1167, despite the presence of the channels 500.

Alternatively, as shown in FIGURE 14E, in some embodiments, the channels 500 on the first surface 1405 may align with the channels 500 on the second surface 1410. In some embodiments, the center or deepest portion of each channel 500 in the first surface 1405 may be aligned with and/or directly across from the center or deepest portion of a corresponding channel 500 in the second surface 1410, so that the channel 500 in the first surface 1405 and the corresponding channel 500 in the second surface 1410 may be stacked and separated across the thickness of the treatment manifold 1167. This alignment may cause the thickness of the treatment manifold 1167 to vary along its length and/or width, for example being thinner at points between aligned channels 500 (e.g. between corresponding channels 500 on the first and second surfaces), and wider at points between adjacent sets of corresponding channels 500 (e.g. as spaced along the treatment manifold 1167). In some embodiments, a line extending perpendicular through the deepest portion of a channel 500 on the first surface 1405 and through the thickness of the treatment manifold 1167, may also extend perpendicularly through the deepest portion of the corresponding channel 500 on the second surface 1410 of the treatment manifold 1167, bisecting corresponding channels 500 on opposite surfaces of the treatment manifold 1167.

In some embodiments, each of the plurality of channels 500 may be approximately 2 mm wide and/or approximately 2 mm deep. In some embodiments, the depth of each of the plurality of channels 500 may be about 1/3-1/2 of the manifold thickness (e.g. in portions of the treatment manifold 1167 without channels 500). In some embodiments, the plurality of channels 500 may cover approximately 50% of the surface area of the first surface 1405 and/or approximately 50% of the surface area of the second surface 1410 of the treatment manifold 1167. In some embodiments, the plurality of channels 500 may only span the first and/or second surface 1410 of the plurality of leg manifold members 1160. In some embodiments, the plurality of channels 500 may also span the central connection manifold member 1154, in addition to the leg manifold members 1160, for example spanning the entire first and/or second surface 1410 of the treatment manifold 1167.

In some embodiments, the plurality of channels 500 may comprise a plurality of longitudinal channels 1430, which may each extend lengthwise along the corresponding leg manifold member 1160, and a plurality of lateral channels 1435, which may extend across the width of the corresponding leg manifold member 1160. In some embodiments, the longitudinal channels 1430 and the lateral channels 1435 may jointly form a grid of channels 500, which may be similar to the grid or grids described with respect to FIGURE 5A. The grid of channels 500 may be configured to effectively distribute negative pressure across the treatment manifold 1167 and/or to effectively draw fluid from across the treatment manifold 1167. In some embodiments, each leg manifold member 1160 may comprise 2-3 longitudinal channels 1430 on the first surface 1405. In some embodiments, each leg manifold member 1160 may comprise 2-3 longitudinal channels 1430 on the second surface 1410. In some embodiments, some of the plurality of longitudinal channels 1430 may be located on the first surface 1405 of the treatment manifold 1167, and some of the plurality of lateral channels 1435 may be located on the first surface 1405 of the treatment manifold 1167, some of the plurality of longitudinal channels 1430 may be located on the second surface 1410 of the treatment manifold 1167, and/or some of the plurality of lateral channels 1435 may be located on the second surface 1410 of the treatment manifold 1167. In some embodiments, both the first surface 1405 and the second surface 1410 of the treatment manifold 1167 may have a grid of channels 500.

In some embodiments, the plurality of through-passages in the treatment manifold 1167 may comprise a plurality of contraction apertures 210 and/or a plurality of through-holes 1425. In some embodiments, the through-holes 1425 may each have a diameter of about 1-2 mm. In some embodiments, the through-holes 1425 may be distributed or located only on the leg manifold members 1160, for example with each through-hole 1425 passing through the thickness of the corresponding leg manifold member 1160. In some embodiments, each through-hole 1425 may intersect at least one channel 500. In some embodiments, each through-hole 1425 may intersect at least one longitudinal channel 1430 and at least one lateral channel 1435 (e.g. the through-hole 1425 may be located with respect to the grid of channels 500 at the intersection of a lateral channel 1435 and a longitudinal channel 1430). In some embodiments, each through-hole 1425 may intersect at least one channel 500 on the first surface 1405 and at least one channel 500 on the second surface 1410 of the treatment manifold 1167. In some embodiments, each through-hole 1425 may intersect one longitudinal channel 1430 and one lateral channel 1435 on the first surface 1405, and one longitudinal channel 1430 and one lateral channel 1435 on the second surface 1410. In some embodiments, at least some of the plurality of through-holes 1425 may be located in the central connection manifold member 1154 (e.g. the plurality of through-holes 1425 may be distributed across the entire treatment manifold 1167).

In some embodiments, the central connection manifold member 1154 may comprise a plurality of contraction apertures 210, which may be similar to those shown in FIGURES 2A-4. For example, in some embodiments, the only through-passages in the central connection manifold member 1154 may be contraction apertures 210 (although in other embodiments, the central connection manifold member 1154 may have both through-holes and contraction apertures 210). In some embodiments, the plurality of contraction apertures 210 may only be located or disposed on or through the central connection manifold member 1154. For example, each contraction aperture 210 may pass through the thickness of the central connection manifold member 1154, from the first surface 1405 to the second surface 1410.

In some embodiments, the plurality of contraction apertures 210 in the central connection manifold member 1154 may be configured to provide contraction radially in all directions upon application of negative pressure. In some embodiment, the leg manifold members 1160 may also have contraction apertures 210, which may be configured to provide only lengthwise contraction of the leg manifold members 1160. In some embodiments, all through-passages in the treatment manifold 1167 may be contraction apertures 210. In other embodiments, the through-passages in the central connection manifold member 1154 may be contraction apertures 210, and the through-passages in the leg manifold members 1160 may be through-holes 1425 (e.g. not configured to encourage contraction of the treatment manifold 1167).

In some embodiments, each of the plurality of contraction apertures 210 may intersect at least one of the plurality of channels 500. In some embodiments, each contraction aperture 210 may intersect at least one longitudinal channel 1430 and at least one lateral channel 1435 (e.g. the contraction aperture 210 may be located with respect to the grid of channels 500 at the intersection of a lateral channel 1435 and a longitudinal channel 1430). In some embodiments, each contraction aperture 210 may intersect at least one channel 500 on the first surface 1405 and at least one channel 500 on the second surface 1410 of the treatment manifold 1167. In some embodiments, each contraction aperture 210 may intersect one longitudinal channel 1430 and one lateral channel 1435 on the first surface 1405, and one longitudinal channel 1430 and one lateral channel 1435 on the second surface 1410.

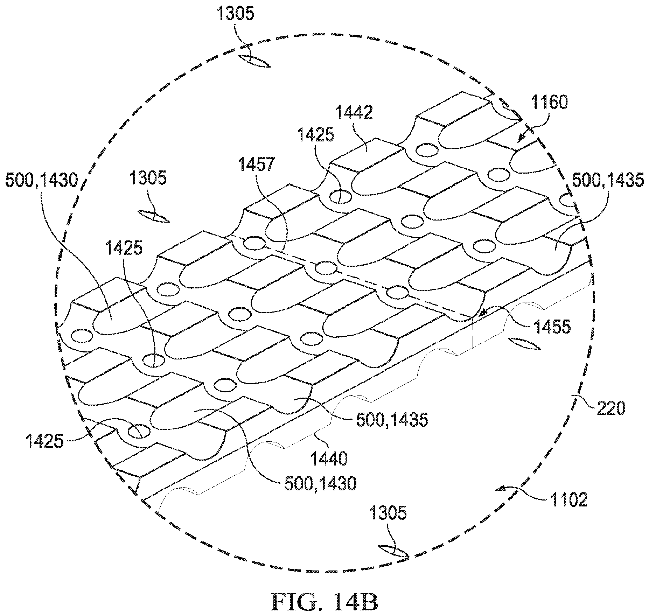

In some embodiments, the film layer 220 may be adhered to the first surface 1405 of the treatment manifold 1167, as shown in FIGURES 14B-C. Although the film layer 220 is shown as being attached to the first surface 1405 of the treatment manifold 1167 in FIGURE 14C, in other embodiments the first surface 1405 and the second surface 1410 may be transposed, so that the film layer 220 may be attached to the second surface 1410. In some embodiments, the film layer 220 may be bonded to the treatment manifold 1167 by an adhesive 905, such as a polyurethane or acrylic adhesive. In some embodiments, the film layer 220 may be similar to the non-adherent drape described with respect to FIGURES 11A and 13A. In some embodiments, the film layer 220 may comprise or consist essentially of polyurethane film. In some embodiments, film layer 220 may be approximately 75 - 120 microns thick. In some embodiments, the film layer 220 may comprise a plurality of fenestrations 1305, perforations, or passages, which may be similar to those described with respect to FIGURES 2B, 11A, and 13A.

In some embodiments, the film layer 220 may span the first surface 1405 of at least the plurality of leg manifold members 1160. In some embodiments, the film layer 220 may span the first surface 1405 of the central connection manifold member 1154. In some embodiments, the film layer 220 may extend between the leg manifold members 1160. In FIGURE 14A, the film layer 220 is shown spanning the first surface 1405 of the entire treatment manifold 1167, and spanning the space between the leg manifold members 1160.

In some embodiments, the film layer 220 may comprises a surface texture (e.g. a micro-texture). In some embodiments, the surface texture may be disposed on one or both of a first film surface 1440 and an opposing, second film surface 1442. For example, as shown in FIGURE 14C, the surface texture may include a plurality of ridges 1445 and grooves 1447, and in some embodiments, at least one of the ridges 1445 on the first film surface 1440 may correspond to a groove 1447 on the second film surface 1442. In some embodiments, each ridge 1445 on the first film surface 1440 may correspond to a groove 1447 on the second film surface 1442, and vice versa. In some embodiments, the surface texture may comprise a recurring pattern forming the textured surface. For example, the surface texture may comprise a pattern of ridges 1445 and grooves 1447, sharp corners, diamonds, triangles, squares, and/or other tessellating shapes, which may form peaks and valleys in the film's surface. In some embodiments, the peak-to-peak height (e.g. overall film thickness, for example between a peak or ridge 1445 on the first film surface 1440 and an adjacent peak or ridge 1445 on the second film surface 1442) may be approximately 1-1.5 mm. In some embodiments, the spacing may be approximately 1-1.5 mm between adjacent peaks or ridges 1445 on the first film surface. In some embodiments, the surface texture may be embossed on the film layer 220. In some embodiments, the surface texture may be imparted to the film layer 220 by heating a film and drawing the heated film over a mandrel tool in order to thermoform the surface texture on the film when the film cools. In other embodiments, the surface texture may be imparted to the film layer 220 by crushing the film through textured rollers.

In some embodiments, the surface texture may be disposed on at least the portion of the film layer 220 between the leg manifold members 1160, which may form additional fluid pathways (e.g. in addition to the channels 500 and/or through-passages in the treatment manifold 1167) for fluid flow when negative pressure is applied to the treatment device 1102. These additional fluid pathways (e.g. formed by the surface texture of the film layer 220) may be configured to allow fluid flow in areas between the leg manifold members 1160 (e.g. providing additional manifolding capabilities, in addition to that provided merely by the treatment manifold 1167). For example, under negative pressure, fluid may flow along the film layer 220 within the grooves 1447 towards the central connection manifold member 1154, and/or fluid may flow along the film layer 220 within the grooves 1447 towards adjacent leg manifold members 1160. In some embodiments, the surface texture may also span portion of the film layer 220 spanning and/or attached to the leg manifold members 1160 and/or the central connection manifold member 1154 (e.g. forming fluid pathways between the film and the treatment manifold 1167).

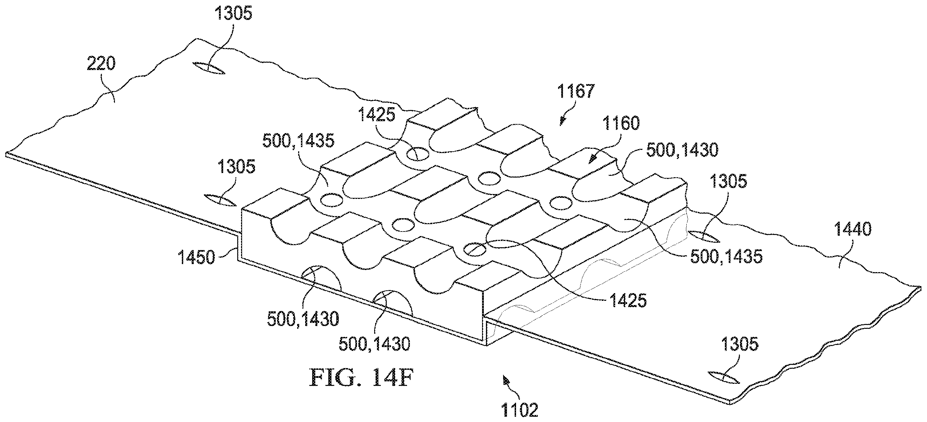

In some embodiments, for example as shown in FIGURE 14F, the film layer 220 may also have one or more indentation 1450 or depression in the first film surface 1440 (e.g. facing the treatment manifold 1167) which may be configured to allow the treatment manifold 1167 to be seated in the film layer 220. Although not shown in FIG. 14F for clarity, the surface texture including the plurality of ridges 1445 and the grooves 1447 as shown in FIGURE 14C may be deployed with the indentation 1450. In some embodiments, the indentation 1450 may be shaped similar to the first surface 1405 of the treatment manifold 1167, such that the indentation 1450 may form an impression of the treatment manifold 1167. In some embodiments, the indentation 1450 may be configured in a complementary shape to at least the first surface 1405 of the treatment manifold 1167, and the indentation 1450 may be configured to allow a complementary portion of the first surface 1405 of the treatment manifold 1167 to be seated in the indentation 1450 in the film layer 220. In some embodiments, when the treatment manifold 1167 is seated within the indentation 1450, the film layer 220 may extend between leg manifold members 1160 at a level or plane located between the first surface 1405 and second surface 1410 of the treatment manifold 1167 (e.g. the depth of the indentation 1450 may be less than the thickness of the treatment manifold 1167).

Some embodiments of the treatment device 1102 may further comprise one or more lines 1455 of sizing perforations 1457 approximately encircling the central connection manifold member 1154. In some embodiments, the lines 1455 of sizing perforations 1457 may form the visual indicia for sizing of the device. In some embodiments, the lines 1455 of sizing perforations 1457 may perforate the film layer 220 and the foam of the leg manifold members, or may perforate the film layer 220 and extend through the manipulation zones of the leg manifold members 1160. In some embodiments, the lines 1455 of sizing perforations 1457 may be configured to allow for tearing or cutting of the treatment device 1102 to appropriately size the treatment device 1102 for a particular patient. In some embodiments, the lines 1455 of sizing perforations 1457 may be spaced apart (e.g. concentrically) approximately 50-60 mm. Sizing of the treatment device 1102 of FIGURES 14A-F (e.g. by tearing or cutting) may not substantially increase the risk of tissue in-growth, since the closed-cell treatment manifold 1167 may resist such tissue in-growth (e.g. despite exposure of the treatment manifold 1167 to the tissue site after tearing or cutting to size the treatment device 1102).

In some embodiments, the closed-cell foam manifold may be coated with starch to form a starch coating (not shown). In some embodiments, the starch coating may be configured to initially restrict contraction of the treatment manifold 1167 and/or to stiffen the treatment manifold 1167, but to break-down over time. For example, the thickness of the starch coating may be approximately 0.5-1 mm. In some embodiments of the treatment device 1102, the treatment manifold 1167 may comprise an antimicrobial coating (not shown). In some embodiments, the antimicrobial coating may comprise silver and/or oxysalts. In some embodiments, the antimicrobial coating may be disposed within the channels 500 of the treatment manifold 1167, for example located between the treatment manifold 1167 and the film layer covering the channels 500 of the first surface 1405 of the treatment manifold 1167.

With respect to manufacturing a dressing or a treatment device, which may be similar to those described above with respect to FIGURES 14A-F, methods may comprise: providing a closed-cell foam treatment manifold (e.g. having a central connection manifold member and a plurality of leg manifold members extending outward from the central connection manifold member, wherein each of the plurality of leg manifold members is in fluid communication with the central connection manifold member, and the closed-cell foam manifold is configured to distribute negative pressure); providing a film layer having a plurality of fenestrations; and attaching the film layer to a first surface of the closed-cell foam treatment manifold, while leaving a second, opposite surface of the closed-cell foam treatment manifold uncovered (e.g. so that the closed-cell foam manifold may be unencapsulated). In some embodiments, attaching the film layer to the closed-cell foam treatment manifold may comprise adhering the film layer to the closed-cell foam treatment manifold. For example, adhering the film layer to the closed cell-foam treatment manifold may comprise applying polyurethane or acrylic adhesive (e.g. applied by a roller, spray coated, or pattern coated) to the first surface of the closed-cell foam treatment manifold; and applying the film layer into contact with the adhesive. The adhesive may be applied so that it is not mobile and may not flow over the film or foam during formation, so that the adhesive may not block the fenestrations in the film layer or the through-passages in the treatment manifold.

In some embodiments, providing the closed-cell-foam treatment manifold may comprise forming the closed-cell foam treatment manifold with a plurality of channels and a plurality of through-passages, for example with each of the through-passages intersecting at least one of the channels. In some embodiments, the plurality of channels may be formed on at least the first surface of the closed-cell foam treatment manifold. In some embodiments, the plurality of channels may be formed on the first surface and the second surface of the closed-cell foam treatment manifold. In some embodiments, the channels on the first surface may be offset from the channels on the second surface, and providing the closed-cell-foam treatment manifold may comprise providing a 4 mm thick foam blank. In some embodiments, the channels on the first surface may be aligned with the channels in the second surface, and providing the closed-cell-foam treatment manifold may comprise providing a 5-6 mm thick foam blank. In some embodiments, at least some of the plurality of channels may be formed to extend along a length of the plurality of leg manifold members. In some embodiments, forming the closed-cell foam treatment manifold may comprise providing a blank of closed-cell foam; and thermoforming the plurality of channels. In some embodiments, forming the closed-cell foam treatment manifold may comprise perforating the foam blank to form the plurality of through-passages. Some method embodiments may further comprise maintaining an exposed second surface of the closed-cell foam treatment manifold (e.g. leaving the second surface of the treatment manifold exposed, so that the finished treatment device may not be encapsulated, but may have at most one film layer attached to the treatment manifold).

In some embodiments, providing the film layer may comprise providing a flat film and forming a surface texture (e.g. micro-texture) on the film. In some embodiments, forming the surface texture may comprise thermoforming the film to form the film layer with recurring texture pattern. In some embodiments, forming the surface texture may comprise crushing the film through textured rollers (e.g. embossing). In some embodiments, providing the film layer may comprise providing a flat film and forming an indentation on the film (e.g. the first film surface of the film layer) configured to allow the closed-cell foam treatment manifold to be seated within the indentation. In some embodiments, forming the indentation may comprise thermoforming the flat film with an impression of the first surface of the treatment manifold. In some embodiments, thermoforming (e.g. to form the surface texture and/or the indentation) may comprise heating the film and drawing the heated film over a mandrel tool. When thermoforming, the film may range in thickness from 75-120 micron.

Some method embodiments may further comprise forming contraction apertures in at least the central connection manifold member. In some method embodiments, the contraction apertures in the central connection manifold member may be formed to produce radial contraction under negative pressure. Some method embodiments may further comprise coating the closed-cell foam treatment manifold with a starch, for example to form a starch coating with a thickness of about 0.5-1 mm). In some embodiments, the method may further comprise selecting and providing the starch to substantially prevent contraction, but to break-down and/or be removed by fluid flow from the tissue site in order to allow significant contraction of at least the legs.

The systems, apparatuses, and methods described herein may provide significant advantages. For example, some embodiments may be configured to allow for resizing, without significant risk of tissue in-growth. Minimizing tissue in-growth may allow for easier removal of the dressing and/or treatment device, without significant damage being caused to the tissue site during removal. Some embodiments may also be configured to allow for the dressing to be left in place during usage for extended periods of time without the need for frequent dressing changes, preventing tissue in-growth that can arise during extended wear. Some embodiments may also be configured to minimize fluid retention in the treatment device, which may prove beneficial in reducing risk of infection. Some embodiments may be configured to provide radial contraction of the treatment device when negative pressure is applied, which may improve fluid and pressure manifolding and/or draw the edges of the wound together to reduce overall wound size. Some embodiments may be configured to provide additional manifolding and fluid flow (e.g. between the legs of the treatment manifold), thereby improving the overall ability of the dressing to remove fluids from the wound. Some embodiments of the treatment device may provide easier construction and may be more economical, for example by minimizing the number of layers and/or by seating and/or attaching the layers to minimize movement during manufacture, which may result in improved pricing for end users. Some embodiments may provide for ease of usage, and may require less training for personnel to effectively size and place the dressing in a wound, for example by allowing sizing of the dressing to the specific wound without significant concern regarding tissue in-growth. These and other advantages may arise from the disclosure.

If something is described as "exemplary" or an "example", it should be understood that refers to a non-exclusive example. The terms "about" or "approximately" or the like, when used with a number, may mean that specific number, or alternatively, a range in proximity to the specific number as understood by persons of skill in the art field (for example, +/-10%). Use of broader terms such as "comprises", "includes", and "having" should be understood to provide support for narrower terms such as "consisting of", "consisting essentially of', and "comprised substantially of'. Use of the term "optionally", "may", "might", "possibly", "could", "can", "would", "should", "preferably", "typically", "often" and the like with respect to any element, component, feature, characteristic, etc. of an embodiment means that the element, component, feature, characteristic, etc. is not required, or alternatively, the element, component, feature, characteristic, etc. is required, both alternatives being within the scope of the embodiment(s). Such element, component, feature, characteristic, etc. may be optionally included in some embodiments, or it may be excluded

Section headings used herein are provided for consistency and convenience, and shall not limit or characterize any invention(s) set out in any claims that may issue from this disclosure. If a reference numeral is used to reference a specific example of a more general term, then that reference numeral may also be used to refer to the general term (or vice versa).

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing, the container, or both may be eliminated or separated from other components for manufacture or sale. In other example configurations, the controller may also be manufactured, configured, assembled, or sold independently of other components.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art.

Also, features, elements, and aspects described with respect to a particular embodiment may be combined with features, elements, and aspects described with respect to one or more other embodiments.

## Claims

1. A treatment device comprising:
a closed-cell foam treatment manifold (1167) having a central connection manifold member (1154) and a plurality of leg manifold members (1160) extending outward from the central connection manifold member (1154), wherein each of the plurality of leg manifold members (1160) is in fluid communication with the central connection manifold member (1154); and
a single film layer (220) disposed in proximity to and attached to a first surface (1405) of the treatment manifold (1167), wherein the film layer (220) comprises a plurality of fenestrations (1305);
wherein a second surface (1410) of the treatment manifold (1167) is uncovered, and the second surface (1410) is opposite the first surface (1405).

2. The treatment device of claim 1, wherein the film layer (220) extends between the leg manifold members (1160).

3. The treatment device of claim 1, wherein the treatment manifold (1167) comprises a plurality of channels (500) and a plurality of through-passages, wherein the plurality of channels (500) are disposed on at least one of the first surface (1405) and a second surface (1410) opposite the first surface (1405), and wherein the plurality of through-passages are disposed through the first surface (1405) and the second surface (1410).

4. The treatment device of claim 3, wherein the plurality of channels (500) are disposed on both the first surface (1405) and the second surface (1410).

5. The treatment device of claim 3, wherein at least some of the through-passages intersect at least some of the channels (500).

6. The treatment device of claim 4, wherein the channels (500) on the first surface (1405) are offset from the channels (500) on the second surface (1410).

7. The treatment device of claim 4, wherein the channels (500 on the first surface (1405) align with the channels on the second surface (1410).

8. The treatment device of claim 1, wherein the film layer (2200 comprises a surface texture on one or both of a first film surface and an opposing second film surface of the film layer (220), wherein the surface texture includes ridges and grooves.

9. The treatment device of claim 1, wherein the film layer (220) comprises at least one indentation configured in a complementary shape to at least the first surface (1405) of the treatment manifold (1167), wherein the at least one indentation allows a complementary portion of the first surface (1405) of the treatment manifold (1167) to be seated in the at least one indentation in the film layer (22).

10. The treatment device of claim 1, wherein the central connection manifold member (1154) comprises a plurality of contraction apertures (210) configured so that the central connection manifold (1154) member contracts radially in a plane under negative-pressure.

## Patentansprüche

1. Eine Behandlungsvorrichtung, aufweisend:
einen Verteiler (1167) für eine Behandlung von geschlossenzelligem Schaum, der ein zentrales Verbindungsverteilerelement (1154) und eine Mehrzahl von Schenkelverteilerelementen (1160) hat, die sich von dem zentralen Verbindungsverteilerelement (1154) nach außen erstrecken, wobei jedes der Mehrzahl von Schenkelverteilerelementen (1160) in Flüssigkeitskommunikation mit dem zentralen Verbindungsverteilerelement (1154) steht; und
eine einzelne Folienschicht (220), die in der Nähe einer ersten Oberfläche (1405) des Behandlungsverteilers (1167) angeordnet und an dieser befestigt ist, wobei die Folienschicht (220) eine Mehrzahl von Fenstern (1305) aufweist;
wobei eine zweite Oberfläche (1410) des Behandlungsverteilers (1167) unbedeckt ist und die zweite Oberfläche (1410) der ersten Oberfläche (1405) gegenüberliegt.

2. Die Behandlungsvorrichtung nach Anspruch 1, wobei sich die Folienschicht (220) zwischen den Schenkelverteilerelementen (1160) erstreckt.

3. Die Behandlungsvorrichtung nach Anspruch 1, wobei der Behandlungsverteiler (1167) eine Mehrzahl von Kanälen (500) und eine Mehrzahl von Durchgängen aufweist, wobei die Mehrzahl von Kanälen (500) auf mindestens einer der ersten Oberfläche (1405) und einer der ersten Oberfläche (1405) gegenüberliegenden zweiten Oberfläche (1410) angeordnet ist, und wobei die Mehrzahl von Durchgängen durch die erste Oberfläche (1405) und die zweite Oberfläche (1410) angeordnet ist.

4. Die Behandlungsvorrichtung nach Anspruch 3, wobei die Mehrzahl von Kanälen (500) sowohl auf der ersten Oberfläche (1405) als auch auf der zweiten Oberfläche (1410) angeordnet ist.

5. Die Behandlungsvorrichtung nach Anspruch 3, wobei mindestens einige der Durchgänge mindestens einige der Kanäle (500) kreuzen.

6. Die Behandlungsvorrichtung nach Anspruch 4, wobei die Kanäle (500) auf der ersten Oberfläche (1405) von den Kanälen (500) auf der zweiten Oberfläche (1410) versetzt sind.

7. Die Behandlungsvorrichtung nach Anspruch 4, wobei die Kanäle (500) auf der ersten Oberfläche (1405) mit den Kanälen auf der zweiten Oberfläche (1410) ausgerichtet sind.

8. Die Behandlungsvorrichtung nach Anspruch 1, wobei die Folienschicht (2200) eine Oberflächenstruktur auf einer oder beiden einer ersten Folienoberfläche und einer gegenüberliegenden zweiten Folienoberfläche der Filmschicht (220) aufweist, wobei die Oberflächenstruktur Erhöhungen und Rillen einschließt.

9. Die Behandlungsvorrichtung nach Anspruch 1, wobei die Folienschicht (220) mindestens eine Vertiefung aufweist, die in einer komplementären Form zu mindestens der ersten Oberfläche (1405) des Behandlungsverteilers (1167) konfiguriert ist, wobei die mindestens eine Vertiefung es einem komplementären Abschnitt der ersten Oberfläche (1405) des Behandlungsverteilers (1167) ermöglicht, in der mindestens einen Vertiefung in der Folienschicht (22) zu sitzen.

10. Die Behandlungsvorrichtung nach Anspruch 1, wobei das zentrale Verbindungsverteilerelement (1154) eine Mehrzahl von Kontraktionsöffnungen (210) aufweist, die so konfiguriert sind, dass sich das zentrale Verbindungsverteilerelement (1154) bei Unterdruck in einer Ebene radial zusammenzieht.

## Revendications

1. Dispositif de traitement, comprenant :
un collecteur de traitement de mousse à cellules fermées (1167) ayant un élément de collecteur de raccordement central (1154) et une pluralité d'éléments de collecteur de pattes (1160) s'étendant vers l'extérieur à partir de l'élément de collecteur de raccordement central (1154), dans lequel chacun des éléments de collecteur de pattes (1160) est en communication fluidique avec l'élément de collecteur de raccordement central (1154) ; et
une seule couche de film (220) disposée à proximité d'une première surface (1405) du collecteur de traitement (1167) et fixée à celle-ci, dans lequel la couche de film (220) comprend une pluralité de fenestrations (1305) ;
dans lequel une seconde surface (1410) du collecteur de traitement (1167) est découverte, et la seconde surface (1410) est opposée à la première surface (1405).

2. Dispositif de traitement selon la revendication 1, dans lequel la couche de film (220) s'étend entre les éléments de collecteur de pattes (1160).

3. Dispositif de traitement selon la revendication 1, dans lequel le collecteur de traitement (1167) comprend une pluralité de canaux (500) et une pluralité de passages, dans lequel la pluralité de canaux (500) est disposée sur au moins une parmi la première surface (1405) et une seconde surface (1410) opposée à la première surface (1405), et dans lequel la pluralité de passages est disposée à travers la première surface (1405) et la seconde surface (1410).

4. Dispositif de traitement selon la revendication 3, dans lequel la pluralité de canaux (500) est disposée à la fois sur la première surface (1405) et sur la seconde surface (1410).

5. Dispositif de traitement selon la revendication 3, dans lequel au moins certains des passages croisent au moins certains des canaux (500).

6. Dispositif de traitement selon la revendication 4, dans lequel les canaux (500) de la première surface (1405) sont décalés par rapport aux canaux (500) de la seconde surface (1410).

7. Dispositif de traitement selon la revendication 4, dans lequel les canaux (500) de la première surface (1405) s'alignent sur les canaux de la seconde surface (1410).

8. Dispositif de traitement selon la revendication 1, dans lequel la couche de film (220) comprend une texture de surface sur une ou les deux d'une première surface de film et d'une seconde surface de film opposée de la couche de film (220), dans lequel la texture de surface comporte des crêtes et des rainures.

9. Dispositif de traitement selon la revendication 1, dans lequel la couche de film (220) comprend au moins une indentation conçue dans une forme complémentaire à au moins la première surface (1405) du collecteur de traitement (1167), dans lequel l'au moins une indentation permet à une partie complémentaire de la première surface (1405) du collecteur de traitement (1167) d'être logée dans l'au moins une indentation de la couche de film (22).

10. Dispositif de traitement selon la revendication 1, dans lequel l'élément de collecteur de raccordement central (1154) comprend une pluralité d'ouvertures de contraction (210) conçues de manière à ce que l'élément de collecteur de raccordement central (1154) se contracte radialement dans un plan sous pression négative.
